# EUROPEAN PATENT SPECIFICATION

(11) **EP 4 319 624 B1**
(45) Date of publication and mention of the grant of the patent: **26.08.2026**
(21) Application number: 22735052.7
(22) Date of filing: 20.05.2022
(51) Int. Cl.: A61B 5/00, A61B 5/0215, A61F 2/24, A61M 27/00

(54) **IMPLANT-ADJACENT SENSOR ANCHORING**
IMPLANTATBENACHBARTE SENSORVERANKERUNG
ANCRAGE DE CAPTEUR ADJACENT À UN IMPLANT

(30) Priority: 21.05.2021 US 202163191534 P; 21.07.2021 US 202163224286 P; 23.07.2021 US 202163225039 P; 26.07.2021 US 202163225689 P; 19.08.2021 US 202163235038 P
(43) Date of publication of application: 14.02.2024
(73) Proprietor: Edwards Lifesciences Corporation, Irvine, CA 92614-5688 (US)
(72) Inventor: MCCONNELL, Steven, Anaheim, CA 92808 (US); POOL, Scott Louis, Irvine, CA 92614 (US); MAHMOUDI, Rani Abdullah, Irvine, CA 92614 (US); AMEFIA, Kokou Anani, Aliso Viejo, CA 92656 (US); VALDEZ, Michael G., Irvine, CA 92614 (US); HINZMAN, Julie Ann, Irvine, CA 92606 (US); CHANG, Arvin T., Irvine, CA 92614 (US); RABBAH, Jean-Pierre Michel, Irvine, CA 92614 (US)
(74) Representative: Venner, Julia Ann
(86) International application number: PCT/US2022/030210
(87) International publication number: WO 2022/246168

(56) References cited:
- WO-A1-2020/123338
- WO-A1-2020/163112
- WO-A1-2021/050589
- WO-A1-2021/086707
- CN-A- 107 233 665
- US-A1- 2014 012 101
- US-A1- 2015 039 071
- US-A1- 2016 045 165

## Description

### RELATED APPLICATIONS

This application claims priority based on United States Provisional Patent Application Serial No. 63/191,534, filed May 21, 2021 and entitled IMPLANT-COUPLED SENSORS; United States Provisional Patent Application Serial No. 63/224,286, filed July 21, 2021 and entitled IMPLANT-ADJACENT SENSOR ANCHORING; United States Provisional Patent Application Serial No. 63/225,039, filed July 23, 2021 and entitled SHUNT BARREL SENSOR IMPLANT ANCHORING; United States Provisional Patent Application Serial No. 63/225,689, filed July 26, 2021 and entitled EMBEDDED SENSOR IMPLANT DEVICES; and United States Provisional Patent Application Serial No. 63/235,038, filed August 19, 2021 and entitled SENSOR IMPLANT DEVICE ANCHORING.

### BACKGROUND

### Field

The present disclosure generally relates to the field of medical implant devices.

### Description of Related Art

Various medical procedures involve the implantation of medical implant devices within the anatomy of the heart. Certain physiological parameters associated with such anatomy, such as fluid pressure, can have an impact on patient health prospects.

WO 2020/123338 describes a sensor implant device comprising a shunt structure comprising a flow path conduit and a plurality of arms configured to secure the shunt structure to a tissue wall, and a pressure sensor device attached to one of other plurality of arms of the shunt structure. WO 2021/086707 describes a sensor-retention structure comprising a sensor-support strut and a means for securing a sensor device to the sensor-support strut. WO 2020/163112 describes a septal closure device comprising a frame comprising one or more tissue anchor features, an occluding membrane and a pressure sensor device attached to the occluding membrane. WO 2021/050589 describes an adjustable interatrial shunting system that selectively controls blood flow between the left atrium and right atrium of a patient.

### SUMMARY

Aspects of the presently claimed invention are set out in the independent claims. Particular embodiments of these aspect are set out in the dependent claims. Any subject matter contained herein that does not fall within the scope of the appended claims is considered as being useful for understanding the invention.

Described herein are one or more methods and/or devices to facilitate monitoring of physiological parameter(s) associated with certain chambers and/or vessels of the heart, such as the left atrium, using one or more sensor implant devices (wherein the methods do not form part of the presently claimed invention).

For purposes of summarizing the disclosure, certain aspects, advantages and novel features have been described. It is to be understood that not necessarily all such advantages may be achieved in accordance with any particular example. Thus, the disclosed examples may be carried out in a manner that achieves or optimizes one advantage or group of advantages as taught herein without necessarily achieving other advantages as may be taught or suggested herein.

### BRIEF DESCRIPTION OF THE DRAWINGS

Various examples are depicted in the accompanying drawings for illustrative purposes and should in no way be interpreted as limiting the scope of the inventions. In addition, various features of different disclosed examples can be combined to form additional examples, which are part of this disclosure. Throughout the drawings, reference numbers may be reused to indicate correspondence between reference elements.
Figure 1 illustrates an example representation of a human heart in accordance with one or more examples.
Figure 2 illustrates example pressure waveforms associated with various chambers and vessels of the heart according to one or more examples.
Figure 3 illustrates a graph showing left atrial pressure ranges.
Figure 4 is a block diagram representing an implant device in accordance with one or more examples.
Figure 5 is a block diagram representing a system for monitoring one or more physiological parameters associated with a patient according to one or more examples.
Figure 6 illustrates an example sensor assembly/device that can be a component of a sensor implant device, in accordance with one or more examples.
Figure 7 illustrates an example shunt/anchor structure which may be configured for attachment to one or more sensor bodies, in accordance with one or more examples.
Figure 8 shows a shunt implant/anchor device/structure implanted in an atrial septum in accordance with one or more examples.
Figure 9 shows a shunt device/structure implanted in a tissue wall between the coronary sinus and the left atrium.
Figures 10A-10D illustrate a sensor implant device comprising one or more anchoring arms configured to at least partially embed into one or more tissue walls, in accordance with one or more examples.
Figures 11A and 11B illustrate another sensor implant device configured to collect measurements related to blood flow within a heart, in accordance with one or more examples.
Figure 12 illustrates another sensor implant device configured to collect measurements related to blood flow within a heart, and accordance with one or more examples.
Figures 13-1 and 13-2 provide a flowchart illustrating a process including one or more steps for delivering one or more implants and/or sensors, in accordance with one or more examples.
Figures 14-1 and 14-2 provide images corresponding to steps of the process of Figures 13-1 and 13-2.

### DETAILED DESCRIPTION

The headings provided herein are for convenience only and do not necessarily affect the scope or meaning of the claimed invention.

Although certain preferred examples and examples are below, inventive subject matter extends beyond the specifically disclosed examples to other alternative examples and/or uses and to modifications and equivalents thereof. Thus, the scope of the claims that may arise herefrom is not limited by any of the particular examples described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain examples; however, the order of description should not be construed to imply that these operations are order dependent. Additionally, the structures, systems, and/or devices described herein may be embodied as integrated components or as separate components. For purposes of comparing various examples, certain aspects and advantages of these examples are described. Not necessarily all such aspects or advantages are achieved by any particular example. Thus, for example, various examples may be carried out in a manner that achieves or optimizes one advantage or group of advantages as taught herein without necessarily achieving other aspects or advantages as may also be taught or suggested herein.

Certain reference numbers are re-used across different figures of the figure set of the present disclosure as a matter of convenience for devices, components, systems, features, and/or modules having features that may be similar in one or more respects. However, with respect to any of the examples disclosed herein, re-use of common reference numbers in the drawings does not necessarily indicate that such features, devices, components, or modules are identical or similar. Rather, one having ordinary skill in the art may be informed by context with respect to the degree to which usage of common reference numbers can imply similarity between referenced subject matter. Use of a particular reference number in the context of the description of a particular figure can be understood to relate to the identified device, component, aspect, feature, module, or system in that particular figure, and not necessarily to any devices, components, aspects, features, modules, or systems identified by the same reference number in another figure. Furthermore, aspects of separate figures identified with common reference numbers can be interpreted to share characteristics or to be entirely independent of one another.

Certain standard anatomical terms of location are used herein to refer to the anatomy of animals, and namely humans, with respect to the preferred examples. Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

The present disclosure relates to systems, devices, and methods for monitoring of one or more physiological parameters of a patient (e.g., blood pressure) using sensor-integrated cardiac shunts and/or other medical implant devices. **In** some implementations, the present disclosure relates to cardiac shunts and/or other cardiac implant devices that incorporate or are associated with pressure sensors or other sensor bodies. The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly. Certain examples are disclosed herein in the context of cardiac implant devices. However, although certain principles disclosed herein are particularly applicable to the anatomy of the heart, it should be understood that sensor implant devices in accordance with the present disclosure may be implanted in, or configured for implantation in, any suitable or desirable anatomy.

While the various sensor bodies described herein may be integrated with the various medical implant devices described herein, the sensor bodies may be separate devices from the medical implant devices. For example, a sensor body may form a breakable and/or releasable connection with a medical implant device. Moreover, sensor bodies described herein may be configured to be delivered separately (e.g., before and/or after) medical implant devices within a heart of a patient. For example, a sensor body may not be attached to a medical implant device during delivery processes (e.g., during delivery through a catheter) of the sensor body and/or medical implant device but may be attached/coupled to the medical implant device following delivery (e.g., following removal from a catheter) to a desired location within the heart. Example delivery locations can include the left atrium, the left atrial appendage, the pulmonary vein, the coronary sinus, and/or the various tissue walls associated with these locations.

**In** some examples, a catheter and/or guidewire used for delivering a sensor body may also be used for delivering a medical implant device. For example, the catheter and/or guidewire may remain within the body following delivery of the sensor body and/or medical implant device for delivery of the remaining device(s).

Some sensor bodies described herein may be configured to be delivered prior to delivery of the medical implant devices described herein. This may advantageously simplify delivery of the sensor bodies and/or medical implant devices and/or may provide for simple imaging the sensor bodies and/or medical implant devices. The sensor bodies may be adjusted as necessary to maximize measurements of the sensor bodies prior to delivery of the medical implant devices. Moreover, delivery of the medical implant devices may be delayed and/or aborted as needed following delivery of the sensor bodies.

Some sensor bodies described herein may be configured to be delivered after delivery of the medical implant devices described herein. This may advantageously simplify delivery of the sensor bodies and/or medical implant devices and/or may provide for simple imaging the sensor bodies and/or medical implant devices. The sensor bodies may be adjusted as necessary to maximize measurements of the sensor bodies. Moreover, the sensor body may be effectively secured to the medical implant devices with minimal risk of dislodgement of the sensor bodies.

### Cardiac Physiology

The anatomy of the heart is described below to assist in the understanding of certain inventive concepts disclosed herein. **In** humans and other vertebrate animals, the heart generally comprises a muscular organ having four pumping chambers, wherein the flow thereof is at least partially controlled by various heart valves, namely, the aortic, mitral (or bicuspid), tricuspid, and pulmonary valves. The valves may be configured to open and close in response to a pressure gradient present during various stages of the cardiac cycle (e.g., relaxation and contraction) to at least partially control the flow of blood to a respective region of the heart and/or to blood vessels (e.g., pulmonary, aorta, etc.).

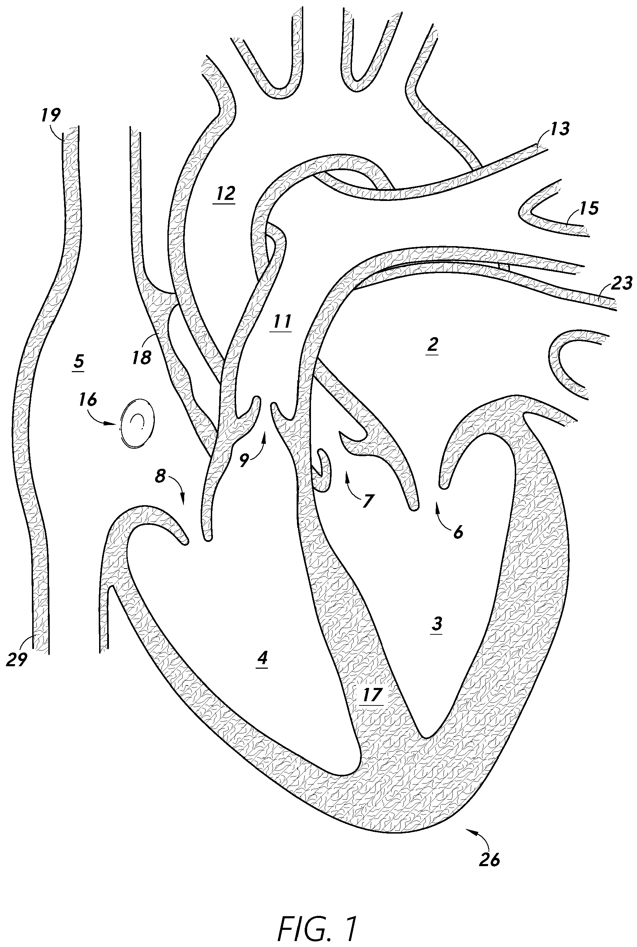

Figure 1 illustrates an example representation of a heart 1 having various features relevant to certain examples of the present inventive disclosure. The heart 1 includes four chambers, namely the left atrium 2, the left ventricle 3, the right ventricle 4, and the right atrium 5. In terms of blood flow, blood generally flows from the right ventricle 4 into the pulmonary artery 11 via the pulmonary valve 9, which separates the right ventricle 4 from the pulmonary artery 11 and is configured to open during systole so that blood may be pumped toward the lungs and close during diastole to prevent blood from leaking back into the heart from the pulmonary artery 11. The pulmonary artery 11 carries deoxygenated blood from the right side of the heart to the lungs. The pulmonary artery 11 includes a pulmonary trunk and left 15 and right 13 pulmonary arteries that branch off of the pulmonary trunk, as shown. The pulmonary veins 23 carry blood from the lungs to the left atrium 2.

In addition to the pulmonary valve 9, the heart 1 includes three additional valves for aiding the circulation of blood therein, including the tricuspid valve 8, the aortic valve 7, and the mitral valve 6. The tricuspid valve 8 separates the right atrium 5 from the right ventricle 4. The tricuspid valve 8 generally has three cusps or leaflets and may generally close during ventricular contraction (i.e., systole) and open during ventricular expansion (i.e., diastole). The mitral valve 6 generally has two cusps/leaflets and separates the left atrium 2 from the left ventricle 3. The mitral valve 6 is configured to open during diastole so that blood in the left atrium 2 can flow into the left ventricle 3, and, when functioning properly, closes during systole to prevent blood from leaking back into the left atrium 2. The aortic valve 7 separates the left ventricle 3 from the aorta 12. The aortic valve 7 is configured to open during systole to allow blood leaving the left ventricle 3 to enter the aorta 12, and close during diastole to prevent blood from leaking back into the left ventricle 3.

The heart valves may generally comprise a relatively dense fibrous ring, referred to herein as the annulus, as well as a plurality of leaflets or cusps attached to the annulus. Generally, the size of the leaflets or cusps may be such that when the heart contracts the resulting increased blood pressure produced within the corresponding heart chamber forces the leaflets at least partially open to allow flow from the heart chamber. As the pressure in the heart chamber subsides, the pressure in the subsequent chamber or blood vessel may become dominant and press back against the leaflets. As a result, the leaflets/cusps come in apposition to each other, thereby closing the flow passage. Dysfunction of a heart valve and/or associated leaflets (e.g., pulmonary valve dysfunction) can result in valve leakage and/or other health complications.

The atrioventricular (i.e., mitral and tricuspid) heart valves may further comprise a collection of chordae tendineae and papillary muscles (not shown) for securing the leaflets of the respective valves to promote and/or facilitate proper coaptation of the valve leaflets and prevent prolapse thereof. The papillary muscles, for example, may generally comprise finger-like projections from the ventricle wall. The valve leaflets are connected to the papillary muscles by the chordae tendineae. A wall of muscle, referred to as the septum, separates the left-side chambers from the right-side chambers. In particular, an atrial septum wall portion 18 (referred to herein as the "atrial septum," "interatrial septum," or "septum") separates the left atrium 2 from the right atrium 5, whereas a ventricular septum wall portion 17 (referred to herein as the "ventricular septum," "interventricular septum," or "septum") separates the left ventricle 3 from the right ventricle 4. The inferior tip 26 of the heart 1 is referred to as the apex and is generally located on or near the midclavicular line, in the fifth intercostal space.

The coronary sinus 16 comprises a collection of veins joined together to form a large vessel that collects blood from the heart muscle (myocardium). The ostium of the coronary sinus, which can be guarded at least in part by a Thebesian valve in some patients, is open to the right atrium 5, as shown. The coronary sinus runs along a posterior aspect of the left atrium 2 and delivers less-oxygenated blood to the right atrium 5. The coronary sinus generally runs transversely in the left atrioventricular groove on the posterior side of the heart.

Any of several access pathways in the heart 1 may be utilized for maneuvering guidewires and catheters in and around the heart 1 to deploy implants and/or devices of the present application. For instance, access may be from above via either the subclavian vein or jugular vein into the superior vena cava (SVC) 19, right atrium 5, and from there into the coronary sinus 16. Alternatively, the access path may start in the femoral vein and through the inferior vena cava (IVC) 14 into the heart 1. Other access routes may also be used, and each can utilize a percutaneous incision through which the guidewire and catheter are inserted into the vasculature, normally through a sealed introducer, and from there the physician can control the distal ends of the devices from outside the body.

### Health Conditions Associated with Cardiac Pressure and Other Parameters

As referenced above, certain physiological conditions or parameters associated with the cardiac anatomy can impact the health of a patient. For example, congestive heart failure is a condition associated with the relatively slow movement of blood through the heart and/or body, which causes the fluid pressure in one or more chambers of the heart to increase. As a result, the heart does not pump sufficient oxygen to meet the body's needs. The various chambers of the heart may respond to pressure increases by stretching to hold more blood to pump through the body or by becoming relatively stiff and/or thickened. The walls of the heart can eventually weaken and become unable to pump as efficiently. In some cases, the kidneys may respond to cardiac inefficiency by causing the body to retain fluid. Fluid build-up in arms, legs, ankles, feet, lungs, and/or other organs can cause the body to become congested, which is referred to as congestive heart failure. Acute decompensated congestive heart failure is a leading cause of morbidity and mortality, and therefore treatment and/or prevention of congestive heart failure is a significant concern in medical care.

The treatment and/or prevention of heart failure (e.g., congestive heart failure) can advantageously involve the monitoring of pressure in one or more chambers or regions of the heart or other anatomy. As described above, pressure buildup in one or more chambers or areas of the heart can be associated with congestive heart failure. Without direct or indirect monitoring of cardiac pressure, it can be difficult to infer, determine, or predict the presence or occurrence of congestive heart failure. For example, treatments or approaches not involving direct or indirect pressure monitoring may involve measuring or observing other present physiological conditions of the patient, such as measuring body weight, thoracic impedance, right heart catheterization, or the like. **In** some solutions, pulmonary capillary wedge pressure can be measured as a surrogate of left atrial pressure. For example, a pressure sensor may be disposed or implanted in the pulmonary artery, and readings associated therewith may be used as a surrogate for left atrial pressure. However, with respect to catheter-based pressure measurement in the pulmonary artery or certain other chambers or regions of the heart, use of invasive catheters may be required to maintain such pressure sensors, which may be uncomfortable or difficult to implement. Furthermore, certain lung-related conditions may affect pressure readings in the pulmonary artery, such that the correlation between pulmonary artery pressure and left atrial pressure may be undesirably attenuated. As an alternative to pulmonary artery pressure measurement, pressure measurements in the right ventricle outflow tract may relate to left atrial pressure as well. However, the correlation between such pressure readings and left atrial pressure may not be sufficiently strong to be utilized in congestive heart failure diagnostics, prevention, and/or treatment.

Additional solutions may be implemented for deriving or inferring left atrial pressure. For example, the E/A ratio, which is a marker of the function of the left ventricle of the heart representing the ratio of peak velocity blood flow from gravity in early diastole (the E wave) to peak velocity flow in late diastole caused by atrial contraction (the A wave), can be used as a surrogate for measuring left atrial pressure. The E/A ratio may be determined using echocardiography or other imaging technology; generally, abnormalities in the E/A ratio may suggest that the left ventricle cannot fill with blood properly in the period between contractions, which may lead to symptoms of heart failure, as explained above. However, E/A ratio determination generally does not provide absolute pressure measurement values.

Various methods for identifying and/or treating congestive heart failure involve the observation of worsening congestive heart failure symptoms and/or changes in body weight. However, such signs may appear relatively late and/or be relatively unreliable. For example, daily bodyweight measurements may vary significantly (e.g., up to 9% or more) and may be unreliable in signaling heart-related complications. Furthermore, treatments guided by monitoring signs, symptoms, weight, and/or other biomarkers have not been shown to substantially improve clinical outcomes. **In** addition, for patients that have been discharged, such treatments may necessitate remote telemedicine systems.

The present disclosure provides systems, devices, and methods for guiding the administration of medication relating to the treatment of congestive heart failure at least in part by directly monitoring pressure in the left atrium, or other chamber or vessel for which pressure measurements are indicative of left atrial pressure and/or pressure levels in one or more other vessels/chambers, such as for congestive heart failure patients in order to reduce hospital readmissions, morbidity, and/or otherwise improve the health prospects of the patient.

### Cardiac Pressure Monitoring

Cardiac pressure monitoring in accordance with examples of the present disclosure may provide a proactive intervention mechanism for preventing or treating congestive heart failure and/or other physiological conditions. Generally, increases in ventricular filling pressures associated with diastolic and/or systolic heart failure can occur prior to the occurrence of symptoms that lead to hospitalization. For example, cardiac pressure indicators may present weeks prior to hospitalization with respect to some patients. Therefore, pressure monitoring systems in accordance with examples of the present disclosure may advantageously be implemented to reduce instances of hospitalization by guiding the appropriate or desired titration and/or administration of medications before the onset of heart failure.

Dyspnea represents a cardiac pressure indicator characterized by shortness of breath or the feeling that one cannot breathe well enough. Dyspnea may result from elevated atrial pressure, which may cause fluid buildup in the lungs from pressure back-up. Pathological dyspnea can result from congestive heart failure. However, a significant amount of time may elapse between the time of initial pressure elevation and the onset of dyspnea, and therefore symptoms of dyspnea may not provide sufficiently-early signaling of elevated atrial pressure. By monitoring pressure directly according to examples of the present disclosure, normal ventricular filling pressures may advantageously be maintained, thereby preventing or reducing effects of heart failure, such as dyspnea.

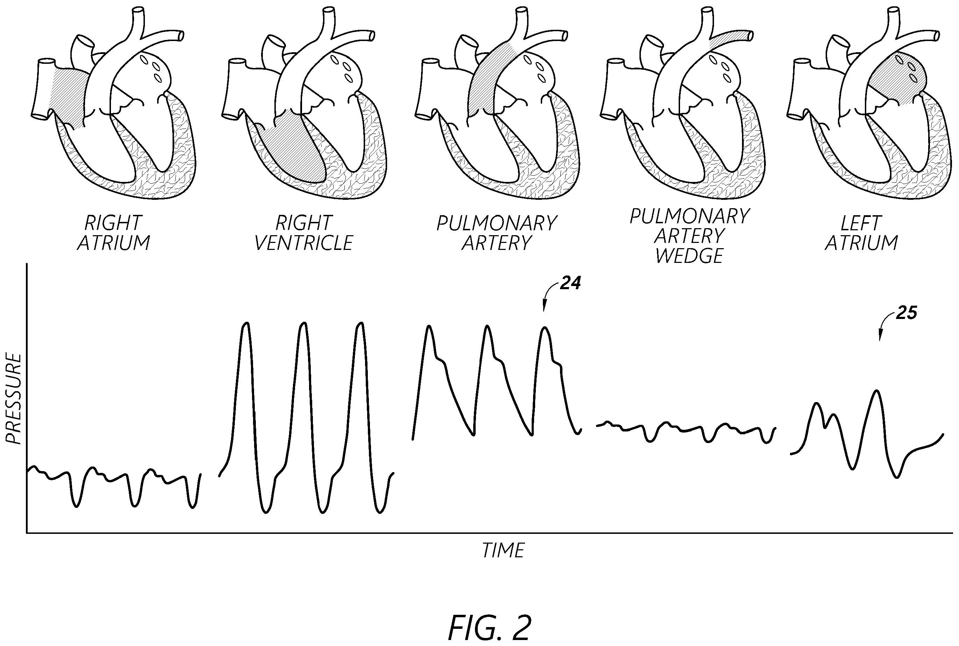

As referenced above, with respect to cardiac pressures, pressure elevation in the left atrium may be particularly correlated with heart failure. Figure 2 illustrates example pressure waveforms associated with various chambers and vessels of the heart according to one or more examples. The various waveforms illustrated in Figure 2 may represent waveforms obtained using right heart catheterization to advance one or more pressure sensors to the respective illustrated and labeled chambers or vessels of the heart. As illustrated in Figure 2, the waveform 25, which represents left atrial pressure, may be considered to provide the best feedback for early detection of congestive heart failure. Furthermore, there may generally be a relatively strong correlation between increases and left atrial pressure and pulmonary congestion.

Left atrial pressure may generally correlate well with left ventricular end-diastolic pressure. However, although left atrial pressure and end-diastolic pulmonary artery pressure can have a significant correlation, such correlation may be weakened when the pulmonary vascular resistance becomes elevated. That is, pulmonary artery pressure generally fails to correlate adequately with left ventricular end-diastolic pressure in the presence of a variety of acute conditions, which may include certain patients with congestive heart failure. For example, pulmonary hypertension, which affects approximately 25% to 83% of patients with heart failure, can affect the reliability of pulmonary artery pressure measurement for estimating left-sided filling pressure. Therefore, pulmonary artery pressure measurement alone, as represented by the waveform 24, may be an insufficient or inaccurate indicator of left ventricular end-diastolic pressure, particularly for patients with comorbidities, such as lung disease and/or thromboembolism. Left atrial pressure may further be correlated at least partially with the presence and/or degree of mitral regurgitation.

Left atrial pressure readings may be relatively less likely to be distorted or affected by other conditions, such as respiratory conditions or the like, compared to the other pressure waveforms shown in Figure 2. Generally, left atrial pressure may be significantly predictive of heart failure, such as up two weeks before manifestation of heart failure. For example, increases in left atrial pressure, and both diastolic and systolic heart failure, may occur weeks prior to hospitalization, and therefore knowledge of such increases may be used to predict the onset of congestive heart failure, such as acute debilitating symptoms of congestive heart failure.

Cardiac pressure monitoring, such as left atrial pressure monitoring, can provide a mechanism to guide administration of medication to treat and/or prevent congestive heart failure. Such treatments may advantageously reduce hospital readmissions and morbidity, as well as provide other benefits. An implanted pressure sensor in accordance with examples of the present disclosure may be used to predict heart failure up two weeks or more before the manifestation of symptoms or markers of heart failure (e.g., dyspnea). When heart failure predictors are recognized using cardiac pressure sensor examples in accordance with the present disclosure, certain prophylactic measures may be implemented, including medication intervention, such as modification to a patient's medication regimen, which may help prevent or reduce the effects of cardiac dysfunction. Direct pressure measurement in the left atrium can advantageously provide an accurate indicator of pressure buildup that may lead to heart failure or other complications. For example, trends of atrial pressure elevation may be analyzed or used to determine or predict the onset of cardiac dysfunction, wherein drug or other therapy may be augmented to cause reduction in pressure and prevent or reduce further complications.

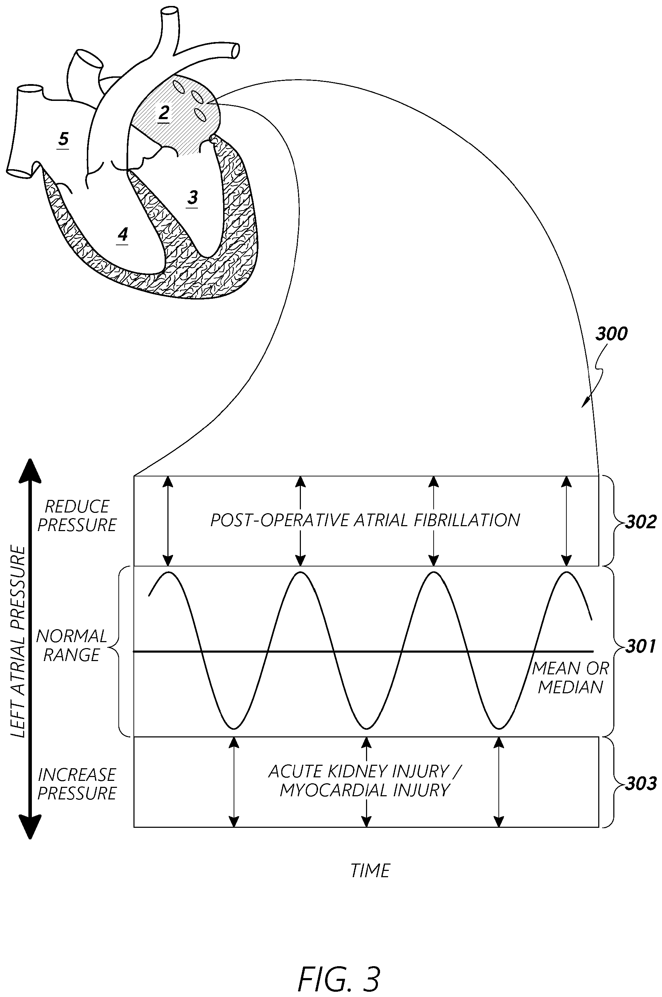

Figure 3 illustrates a graph 300 showing left atrial pressure ranges including a normal range 301 of left atrial pressure that is not generally associated with substantial risk of postoperative atrial fibrillation, acute kidney injury, myocardial injury, heart failure and/or other health conditions. Examples of the present disclosure provide systems, devices, and methods for determining whether a patient's left atrial pressure is within the normal range 301, above the normal range 303, or below the normal range 302 through the use of certain sensor implant devices. For detected left atrial pressure above the normal range, which may be correlated with an increased risk of heart failure, examples of the present disclosure as described in detail below can inform efforts to reduce the left atrial pressure until it is brought within the normal range 301. Furthermore, for detected left atrial pressure that is below the normal range 301, which may be correlated with increased risks of acute kidney injury, myocardial injury, and/or other health complications, examples of the present disclosure as described in detail below can serve to facilitate efforts to increase the left atrial pressure to bring the pressure level within the normal range 301.

### Implant Devices with Integrated Sensors

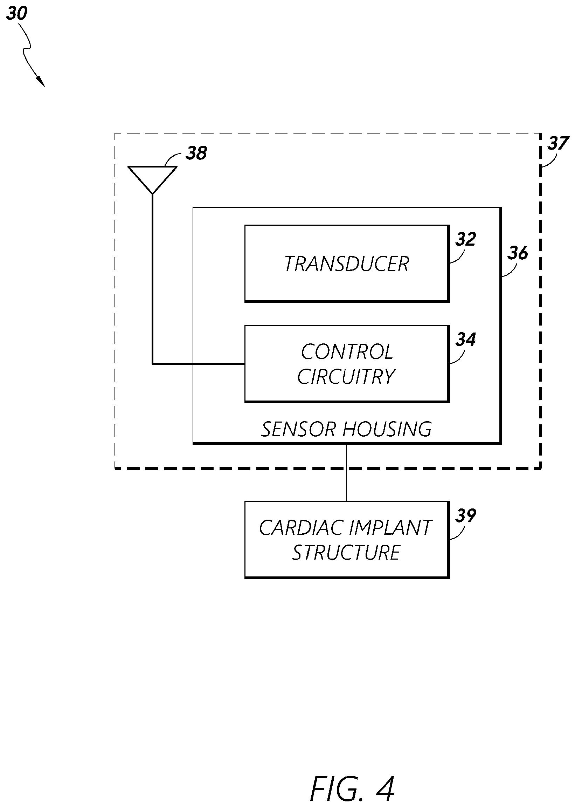

In some implementations, the present disclosure relates to sensors associated or integrated with cardiac shunts or other implant devices. Such integrated devices may be used to provide controlled and/or more effective therapies for treating and preventing heart failure and/or other health complications related to cardiac function. Figure 4 is a block diagram illustrating an implant device 30 comprising a shunt (or other type of implant) structure 39. In some examples, the shunt structure 39 is physically integrated with and/or connected to a sensor body 37. The sensor body 37 may be, for example, a pressure sensor, or other type of sensor. In some examples, the sensor 37 comprises a transducer 32, such as a pressure transducer, as well as certain control circuitry 34, which may be embodied in, for example, an application-specific integrated circuit (ASIC).

The control circuitry 34 may be configured to process signals received from the transducer 32 and/or communicate signals associated therewith wirelessly through biological tissue using the antenna 38. The term "control circuitry" is used herein according to its broad and ordinary meaning, and may refer to any collection of processors, processing circuitry, processing modules/units, chips, dies (e.g., semiconductor dies including come or more active and/or passive devices and/or connectivity circuitry), microprocessors, microcontrollers, digital signal processors, microcomputers, central processing units, field programmable gate arrays, programmable logic devices, state machines (e.g., hardware state machines), logic circuitry, analog circuitry, digital circuitry, and/or any device that manipulates signals (analog and/or digital) based on hard coding of the circuitry and/or operational instructions. Control circuitry referenced herein may further comprise one or more, storage devices, which may be embodied in a single memory device, a plurality of memory devices, and/or embedded circuitry of a device. Such data storage may comprise read-only memory, random access memory, volatile memory, non-volatile memory, static memory, dynamic memory, flash memory, cache memory, data storage registers, and/or any device that stores digital information. It should be noted that in examples in which control circuitry comprises a hardware and/or software state machine, analog circuitry, digital circuitry, and/or logic circuitry, data storage device(s)/register(s) storing any associated operational instructions may be embedded within, or external to, the circuitry comprising the state machine, analog circuitry, digital circuitry, and/or logic circuitry. The transducer(s) 32 and/or antenna(s) 38 can be considered part of the control circuitry 34.

The antenna 38 may comprise one or more coils or loops of conductive material, such as copper wire or the like. In some examples, at least a portion of the transducer 32, control circuitry 34, and/or the antenna 38 are at least partially disposed or contained within a sensor housing 36, which may comprise any type of material, and may advantageously be at least partially hermetically sealed. For example, the housing 36 may comprise glass or other rigid material in some examples, which may provide mechanical stability and/or protection for the components housed therein. In some examples, the housing 36 is at least partially flexible. For example, the housing may comprise polymer or other flexible structure/material, which may advantageously allow for folding, bending, or collapsing of the sensor 37 to allow for transportation thereof through a catheter or other introducing means.

The transducer 32 may comprise any type of sensor means or mechanism. For example, the transducer 32 may be a force-collector-type pressure sensor. In some examples, the transducer 32 comprises a diaphragm, piston, bourdon tube, bellows, or other strain- or deflection-measuring component(s) to measure strain or deflection applied over an area/surface thereof. The transducer 32 may be associated with the housing 36, such that at least a portion thereof is contained within or attached to the housing 36. With respect to sensor bodies/components being "associated with" a stent or other implant structure, such terminology may refer to a sensor body or component being physically coupled, attached, or connected to, integrated with, and/or adjacent to the implant structure.

In some examples, the transducer 32 comprises or is a component of a piezoresistive strain gauge, which may be configured to use a bonded or formed strain gauge to detect strain due to applied pressure, wherein resistance increases as pressure deforms the component/material. The transducer 32 may incorporate any type of material, including but not limited to silicon (e.g., monocrystalline), polysilicon thin film, bonded metal foil, thick film, silicon-on-sapphire, sputtered thin film, and/or the like.

In some examples, the transducer 32 comprises or is a component of a capacitive pressure sensor including a diaphragm and pressure cavity configured to form a variable capacitor to detect strain due to pressure applied to the diaphragm. The capacitance of the capacitive pressure sensor may generally decrease as pressure deforms the diaphragm. The diaphragm may comprise any material(s), including but not limited to metal, ceramic, silicon, and the like. **In** some examples, the transducer 32 comprises or is a component of an electromagnetic pressure sensor, which may be configured to measure the displacement of a diaphragm by means of changes in inductance, linear variable displacement transducer (LVDT) functionality, Hall Effect, or eddy current sensing. **In** some examples, the transducer 32 comprises or is a component of a piezoelectric strain sensor. For example, such a sensor may determine strain (e.g., pressure) on a sensing mechanism based on the piezoelectric effect in certain materials, such as quartz.

**In** some examples, the transducer 32 comprises or is a component of a strain gauge. For example, a strain gauge example may comprise a pressure sensitive element on or associated with an exposed surface of the transducer 32. **In** some examples, a metal strain gauge is adhered to a surface of the sensor, or a thin-film gauge may be applied on the sensor by sputtering or other technique. The measuring element or mechanism may comprise a diaphragm or metal foil. The transducer 32 may comprise any other type of sensor or pressure sensor, such as optical, potentiometric, resonant, thermal, ionization, or other types of strain or pressure sensors.

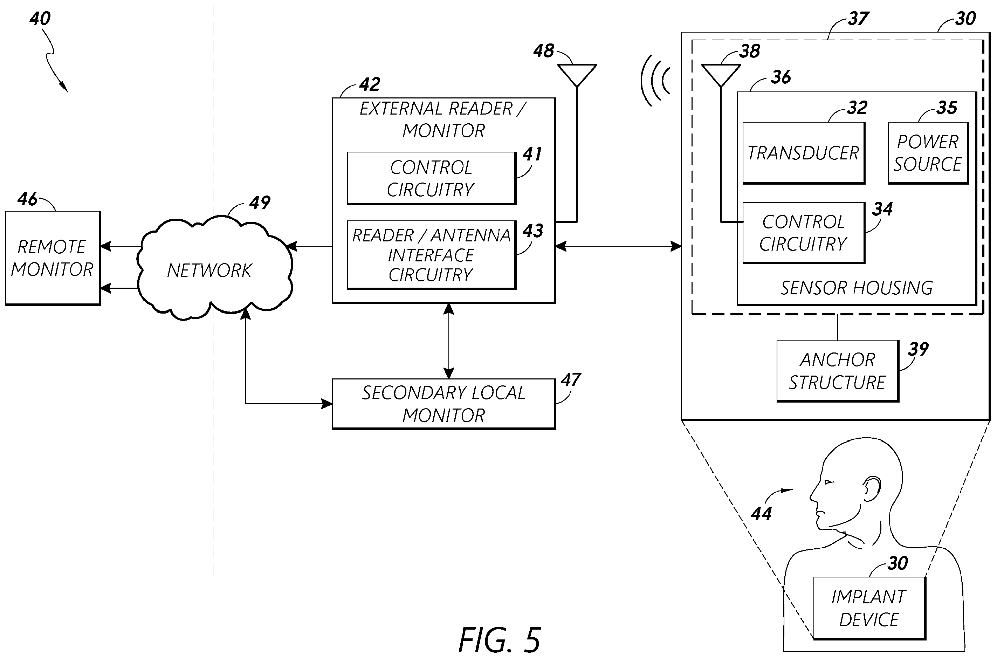

Figure 5 shows a system 40 for monitoring one or more physiological parameters (e.g., left atrial pressure and/or volume) in a patient 44 according to one or more examples. The patient 44 can have a medical implant device 30 implanted in, for example, the heart (not shown), or associated physiology, of the patient 44. For example, the implant device 30 can be implanted at least partially within the left atrium and/or coronary sinus of the patient's heart. The implant device 30 can include one or more sensor transducers 32, such as one or more microelectromechanical system (MEMS) devices (e.g., MEMS pressure sensors, or other type of sensor transducer).

**In** certain examples, the monitoring system 40 can comprise at least two subsystems, including an implantable internal subsystem or device 30 that includes the sensor transducer(s) 32, as well as control circuitry 34 comprising one or more microcontroller(s), discrete electronic component(s), and one or more power and/or data transmitter(s) 38 (e.g., antennae coil). The monitoring system 40 can further include an external (e.g., non-implantable) subsystem that includes an external reader 42 (e.g., coil), which may include a wireless transceiver that is electrically and/or communicatively coupled to certain control circuitry 41. **In** certain examples, both the internal 30 and external 42 subsystems include a corresponding coil antenna for wireless communication and/or power delivery through patient tissue disposed therebetween. The sensor implant device 30 can be any type of implant device. For example, in some examples, the implant device 30 comprises a pressure sensor integrated with another functional implant structure 39, such as a prosthetic shunt or stent device/structure.

Certain details of the implant device 30 are illustrated in the enlarged block 30 shown. The implant device 30 can comprise an implant/anchor structure 39 as described herein. For example, the implant/anchor structure 39 can include a percutaneously-deliverable shunt device configured to be secured to and/or in a tissue wall to provide a flow path between two chambers and/or vessels of the heart, as described in detail throughout the present disclosure. Although certain components are illustrated in Figure 5 as part of the implant device 30, it should be understood that the sensor implant device 30 may only comprise a subset of the illustrated components/modules and can comprise additional components/modules not illustrated. The implant device may represent an example of the implant device shown in Figure 4, and vice versa. The implant device 30 can advantageously include one or more sensor transducers 32, which can be configured to provide a response indicative of one or more physiological parameters of the patient 44, such as atrial pressure. Although pressure transducers are described, the sensor transducer(s) 32 can comprise any suitable or desirable types of sensor transducer(s) for providing signals relating to physiological parameters or conditions associated with the implant device 30 and/or patient 44.

The sensor transducer(s) 32 can comprise one or more MEMS sensors, optical sensors, piezoelectric sensors, electromagnetic sensors, strain sensors/gauges, accelerometers, gyroscopes, diaphragm-based sensors, and/or other types of sensors, which can be positioned in the patient 44 to sense one or more parameters relevant to the health of the patient. The transducer 32 may be a force-collector-type pressure sensor. In some examples, the transducer 32 comprises a diaphragm, piston, bourdon tube, bellows, or other strain- or deflection-measuring component(s) to measure strain or deflection applied over an area/surface thereof. The transducer 32 may be associated with the sensor housing 36, such that at least a portion thereof is contained within, or attached to, the housing 36.

In some examples, the transducer 32 comprises or is a component of a strain gauge, which may be configured to use a bonded or formed strain gauge to detect strain due to applied pressure. For example, the transducer 32 may comprise or be a component of a piezoresistive strain gauge, wherein resistance increases as pressure deforms the component/material of the strain gauge. The transducer 32 may incorporate any type of material, including but not limited to silicone, polymer, silicon (e.g., monocrystalline), polysilicon thin film, bonded metal foil, thick film, silicon-on-sapphire, sputtered thin film, and/or the like. In some examples, a metal strain gauge is adhered to the sensor surface, or a thin-film gauge may be applied on the sensor by sputtering or other technique. The measuring element or mechanism may comprise a diaphragm or metal foil. The transducer 32 may comprise any other type of sensor or pressure sensor, such as optical, potentiometric, resonant, thermal, ionization, or other types of strain or pressure sensors.

In some examples, the transducer 32 comprises or is a component of a capacitive pressure sensor including a diaphragm and pressure cavity configured to form a variable capacitor to detect strain due to pressure applied to the diaphragm. The capacitance of the capacitive pressure sensor may generally decrease as pressure deforms the diaphragm. The diaphragm may comprise any material(s), including but not limited to metal, ceramic, silicone, silicon or other semiconductor, and the like. In some examples, the transducer 32 comprises or is a component of an electromagnetic pressure sensor, which may be configured to measures the displacement of a diaphragm by means of changes in inductance, linear variable displacement transducer (LVDT) functionality, Hall Effect, or eddy current sensing. In some examples, the transducer 32 comprises or is a component of a piezoelectric strain sensor. For example, such a sensor may determine strain (e.g., pressure) on a sensing mechanism based on the piezoelectric effect in certain materials, such as quartz.

In some examples, the transducer(s) 32 is/are electrically and/or communicatively coupled to the control circuitry 34, which may comprise one or more application-specific integrated circuit (ASIC) microcontrollers or chips. The control circuitry 34 can further include one or more discrete electronic components, such as tuning capacitors, resistors, diodes, inductors, or the like.

In certain examples, the sensor transducer(s) 32 can be configured to generate electrical signals that can be wirelessly transmitted to a device outside the patient's body, such as the illustrated local external monitor system 42. In order to perform such wireless data transmission, the implant device 30 can include radio frequency (RF) (or other frequency band) transmission circuitry, such as signal processing circuitry and an antenna 38. The antenna 38 can comprise an antenna coil implanted within the patient. The control circuitry 34 may comprise any type of transceiver circuitry configured to transmit an electromagnetic signal, wherein the signal can be radiated by the antenna 38, which may comprise one or more conductive wires, coils, plates, or the like. The control circuitry 34 of the implant device 30 can comprise, for example, one or more chips or dies configured to perform some amount of processing on signals generated and/or transmitted using the device 30. However, due to size, cost, and/or other constraints, the implant device 30 may not include independent processing capability in some examples.

The wireless signals generated by the implant device 30 can be received by the local external monitor device or subsystem 42, which can include a reader/antenna-interface circuitry module 43 configured to receive the wireless signal transmissions from the implant device 30, which is disposed at least partially within the patient 44. For example, the module 43 may include transceiver device(s)/circuitry.

The external local monitor 42 can receive the wireless signal transmissions from the implant device 30 and/or provide wireless power to the implant device 30 using an external antenna 48, such as a wand device. The reader/antenna-interface circuitry 43 can include radio-frequency (RF) (or other frequency band) front-end circuitry configured to receive and amplify the signals from the implant device 30, wherein such circuitry can include one or more filters (e.g., band-pass filters), amplifiers (e.g., low-noise amplifiers), analog-to-digital converters (ADC) and/or digital control interface circuitry, phase-locked loop (PLL) circuitry, signal mixers, or the like. The reader/antenna-interface circuitry 43 can further be configured to transmit signals over a network 49 to a remote monitor subsystem or device 46. The RF circuitry of the reader/antenna-interface circuitry 43 can further include one or more of digital-to-analog converter (DAC) circuitry, power amplifiers, low-pass filters, antenna switch modules, antennas or the like for treatment/processing of transmitted signals over the network 49 and/or for receiving signals from the implant device 30. In certain examples, the local monitor 42 includes control circuitry 41 for performing processing of the signals received from the implant device 30. The local monitor 42 can be configured to communicate with the network 49 according to a known network protocol, such as Ethernet, Wi-Fi, or the like. In certain examples, the local monitor 42 comprises a smartphone, laptop computer, or other mobile computing device, or any other type of computing device.

In certain examples, the implant device 30 includes some amount of volatile and/or non-volatile data storage. For example, such data storage can comprise solid-state memory utilizing an array of floating-gate transistors, or the like. The control circuitry 34 may utilize data storage for storing sensed data collected over a period of time, wherein the stored data can be transmitted periodically to the local monitor 42 or another external subsystem. In certain examples, the implant device 30 does not include any data storage. The control circuitry 34 may be configured to facilitate wireless transmission of data generated by the sensor transducer(s) 32, or other data associated therewith. The control circuitry 34 may further be configured to receive input from one or more external subsystems, such as from the local monitor 42, or from a remote monitor 46 over, for example, the network 49. For example, the implant device 30 may be configured to receive signals that at least partially control the operation of the implant device 30, such as by activating/deactivating one or more components or sensors, or otherwise affecting operation or performance of the implant device 30.

The one or more components of the implant device 30 can be powered by one or more power sources 35. Due to size, cost and/or electrical complexity concerns, it may be desirable for the power source 35 to be relatively minimalistic in nature. For example, high-power driving voltages and/or currents in the implant device 30 may adversely affect or interfere with operation of the heart or other body part associated with the implant device. In certain examples, the power source 35 is at least partially passive in nature, such that power can be received from an external source wirelessly by passive circuitry of the implant device 30, such as through the use of short-range, or near-field wireless power transmission, or other electromagnetic coupling mechanism. For example, the local monitor 42 may serve as an initiator that actively generates an RF field that can provide power to the implant device 30, thereby allowing the power circuitry of the implant device to take a relatively simple form factor. In certain examples, the power source 35 can be configured to harvest energy from environmental sources, such as fluid flow, motion, or the like. Additionally or alternatively, the power source 35 can comprise a battery, which can advantageously be configured to provide enough power as needed over the monitoring period (e.g., 3, 5, 10, 20, 30, 40, or 90 days, or other period of time).

In some examples, the local monitor device 42 can serve as an intermediate communication device between the implant device 30 and the remote monitor 46. The local monitor device 42 can be a dedicated external unit designed to communicate with the implant device 30. For example, the local monitor device 42 can be a wearable communication device, or other device that can be readily disposed in proximity to the patient 44 and implant device 30. The local monitor device 42 can be configured to continuously, periodically, or sporadically interrogate the implant device 30 in order to extract or request sensor-based information therefrom. In certain examples, the local monitor 42 comprises a user interface, wherein a user can utilize the interface to view sensor data, request sensor data, or otherwise interact with the local monitor system 42 and/or implant device 30.

The system 40 can include a secondary local monitor 47, which can be, for example, a desktop computer or other computing device configured to provide a monitoring station or interface for viewing and/or interacting with the monitored cardiac pressure data. In an example, the local monitor 42 can be a wearable device or other device or system configured to be disposed in close physical proximity to the patient and/or implant device 30, wherein the local monitor 42 is primarily designed to receive/transmit signals to and/or from the implant device 30 and provide such signals to the secondary local monitor 47 for viewing, processing, and/or manipulation thereof. The external local monitor system 42 can be configured to receive and/or process certain metadata from or associated with the implant device 30, such as device ID or the like, which can also be provided over the data coupling from the implant device 30.

The remote monitor subsystem 46 can be any type of computing device or collection of computing devices configured to receive, process and/or present monitor data received over the network 49 from the local monitor device 42, secondary local monitor 47, and/or implant device 30. For example, the remote monitor subsystem 46 can advantageously be operated and/or controlled by a healthcare entity, such as a hospital, doctor, or other care entity associated with the patient 44. Although certain examples disclosed herein describe communication with the remote monitor subsystem 46 from the implant device indirectly through the local monitor device 42, in certain examples, the implant device 30 can comprise a transmitter capable of communicating over the network 49 with the remote monitor subsystem 46 without the necessity of relaying information through the local monitor device 42.

In some examples, at least a portion of the transducer 32, control circuitry 34, power source 35 and/or the antenna 38 are at least partially disposed or contained within the sensor housing 36, which may comprise any type of material, and may advantageously be at least partially hermetically sealed. For example, the housing 36 may comprise glass or other rigid material in some examples, which may provide mechanical stability and/or protection for the components housed therein. In some examples, the housing 36 is at least partially flexible. For example, the housing may comprise polymer or other flexible structure/material, which may advantageously allow for folding, bending, or collapsing of the sensor 37 to allow for transportation thereof through a catheter or other percutaneous introducing means.

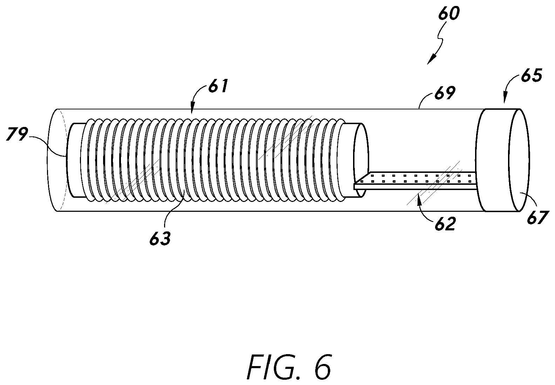

As referenced above, shunt and other implant devices/structures may be integrated with sensor, antenna/transceiver, and/or other components to facilitate in vivo monitoring of pressure and/or other physiological parameter(s). Sensor bodies in accordance with examples of the present disclosure may be integrated with cardiac shunt structures/devices or other implant devices using any suitable or desirable attachment or integration mechanism or configuration. Figure 6 illustrates an example sensor assembly/device 60 that can be a component of a sensor implant device. The sensor body 60 may be configured to provide sensor readings relating to one or more physiological parameters associated with a target implantation site.

The sensor body 60 may be configured for attachment to implant devices and/or tissue walls. For example, a coil form including one or more wires or other material or structure shaped into one or more winds of coil forming a fluid conduit/barrel portion and axial end flanges may be used to attach the sensor body 60 to one or more implants. A shunt structure may be integrated with pressure sensor functionality in accordance with certain examples disclosed herein. The shunt structure may be configured to hold the sensor body 60.

The sensor body 60 may advantageously be disposed, positioned, secured, oriented, and/or otherwise situated in a configuration in which a sensor transducer component 65 thereof is disposed within a channel area of a shunt structure. The term "channel area" is used herein according to its broad and ordinary meaning and may refer to a three-dimensional space defined by a radial boundary of a fluid conduit and extending axially from the fluid conduit.

In some examples, the sensor assembly 61 includes a sensor component 65 and an antenna component 69. The sensor component 65 may comprise any type of sensor body as described in detail above. In some examples, the sensor 65 may be attached to or integrated with an arm member of a shunt structure.

The sensor 65 includes a sensor element 67, such as a pressure sensor transducer. As described herein, the sensor assembly 61 may be configured to implement wireless data and/or power transmission. The sensor assembly 61 may include an antenna component 69 for such purpose. The antenna 69 may be contained at least partially within an antenna housing 79, which may further have disposed therein certain control circuitry configured to facilitate wireless data and/or power communication functionality. In some examples, the antenna component 69 comprises one or more conductive coils 62, which may facilitate inductive powering and/or data transmission. In examples comprising conductive coil(s), such coil(s) may be wrapped/disposed at least partially around a magnetic (e.g., ferrite, iron) core 63.

The antenna component 69 may be attached to, integrated with, or otherwise associated with an arm/anchor feature of a shunt structure

The sensor assembly 61 may advantageously be biocompatible. For example, the sensor 65 and antenna 69 may comprise biocompatible housings, such as a housing comprising glass or other biocompatible material. However, at least a portion of the sensor element 67, such as a diaphragm or other component, may be exposed to the external environment in some examples in order to allow for pressure readings, or other parameter sensing, to be implemented. With respect to the antenna housing 79, the housing 79 may comprise an at least partially rigid cylindrical or tube-like form, such as a glass cylinder form. In some examples, the sensor 65/67 component is approximately 3 mm or less in diameter. The antenna 69 may be approximately 20 mm or less in length.

The sensor assembly 61 may be configured to communicate with an external system when implanted in a heart or other area of a patient's body. For example, the antenna 69 may receive power wirelessly from the external system and/or communicate sensed data or waveforms to and/or from the external system. The sensor assembly 61 may be attached to, or integrated with, a shunt structure in any suitable or desirable way. For example, in some implementations, the sensor 65 and/or antenna 69 may be attached or integrated with the shunt structure using mechanical attachment means. In some examples, the sensor 65 and/or antenna 69 may be contained in a pouch or other receptacle that is attached to a shunt structure.

The sensor element 67 may comprise a pressure transducer. For example, the pressure transducer may be a microelectromechanical system (MEMS) transducer comprising a semiconductor diaphragm component. In some examples, the transducer may include an at least partially flexible or compressible diaphragm component, which may be made from silicone or other flexible material. The diaphragm component may be configured to be flexed or compressed in response to changes in environmental pressure.

### Cardiac Implants

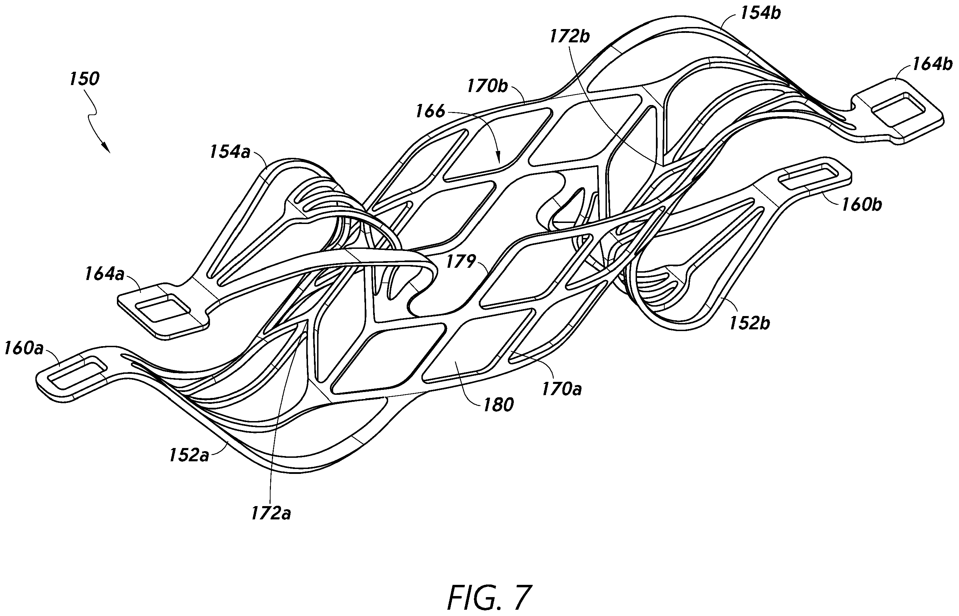

Figure 7 illustrates an example shunt/anchor structure 150 which may be configured for attachment to one or more sensor bodies, in accordance with one or more examples. The shunt structure 150 may represent an example of a cardiac implant (e.g., anchor and/or cardiac implant structure associated with Figure 4 or 5) that may be integrated with pressure sensor functionality in accordance with certain examples disclosed herein. The shunt structure 150 may be an expandable shunt. When expanded, a central flow channel 166 of the shunt 150 may define a generally circular or oval-shaped opening and/or may form a fluid conduit when positioned within an orifice of a tissue wall. The channel 166 may be configured to hold the sides of a puncture opening and/or other orifice in a tissue wall to form a blood flow path between chamber(s) or vessel(s) of the heart that are separated by the tissue wall. For example, the shunt 150 may be configured to be implanted in the wall separating the coronary sinus and the left atrium to form a fluid conduit between the coronary sinus and the left atrium. The central flow channel 166 may be partly formed by a pair of side walls 170a, 170b defined by a generally parallel arrangement of thin struts 179 that forms an array of parallelogram-shaped cells or openings 180. In some examples, substantially the entire shunt 150 is formed by super-elastic struts that are configured to be compressed and fit into a catheter (not shown) and subsequently expanded back to the relaxed shape as shown in Figure 7.

Formation of the shunt 150 using a plurality of interconnected struts forming cells therebetween may serve to at least partially increase the flexibility of the shunt, thereby enabling compression thereof and expansion at the implant site. The interconnected struts around the central flow channel 166 advantageously provide a cage having sufficient rigidity and structure to hold the tissue at the puncture in an open position. End walls 172a, 172b of the central flow channel 166 can serve to connect the side walls 170a, 170b and extend between distal and proximal flanges, or arms, 152, 154 on each side. The side walls 170a, 170b and end walls 172a, 172b together may define a tubular lattice, as shown. The end walls 172a, 172b can comprise thin struts 179 extending at a slight angle from a central flow axis of the shunt 150. The shunt 150 can further comprise terminal ends (160a, 164a, 160b, 164b) of the arms 152, 154 which can be closer together than the ends connected to end walls 172a, 172b of the arms 152, 154.

Although the illustrated shunt 150 comprises struts that define a tubular or circular lattice of open cells forming the central flow channel 166, in some examples, the structure that makes up the channel forms a substantially contiguous wall surface through at least a portion of the channel 166. In the illustrated example, the tilt of the shunt structure 150 may facilitate collapse of the shunt into a delivery catheter (not shown), as well as the expansion of the flanges/arm 152, 154 on both sides of a target tissue wall. The shunt 150 may comprise a first left arm 152a, a second left arm 154a, a first right arm 152b, and/or a second right arm 154b. The central flow channel 166 may remain essentially unchanged between the collapsed and expanded states of the shunt 150, whereas the flanges/arms 152, 154 may transition in and out of alignment with the angled flow channel.

The various struts forming the central flow channel 166 may be configured to contact and/or press against an inner/inside surface of the orifice and/or puncture opening of the tissue wall. For example, a tissue wall may be punctured to create an orifice and/or opening extending at least partially through the tissue wall and/or entirely through the tissue wall. The orifice may represent a fluid conduit between chambers and/or blood flow pathways on either side of the tissue wall. The tissue wall may have any thickness and the orifice may have an inside surface (i.e., radially-inward-facing tissue surface). The shunt 150 may be configured and/or sized such that at least a portion of the central flow channel 166 extends and/or exerts force at least partially along and/or along the entire inside surface of the orifice.

Although certain examples of shunts disclosed herein comprise flow channels and/or fluid conduits having substantially circular cross-sections, in some examples, shunt structures in accordance with the present disclosure have oval-shaped, rectangular, diamond-shaped, or elliptical flow channel configuration. For example, relatively elongated side walls compared to the illustrated configuration of Figure 5 may produce a rectangular or oval-shaped flow channel. Such shapes of shunt flow channels may be desirable for larger punctures, while still being configured to collapse down to a relatively small delivery profile.

In some examples, each of the distal and proximal flanges/arms 152, 154 is configured to curl outward from the end walls 172a, 172b and be set to point approximately radially away from the central flow channel 166 in the expanded configuration. The expanded flanges/arms may serve to secure the shunt 150 to a target tissue wall. Additional aspects and features of shunt, implant, and/or anchor structures that may be integrated with sensor bodies/functionality of examples of the present disclosure are disclosed in U.S. Pat. No. 9,789,294, entitled "Expandable Cardiac Shunt," issued on October 17, 2017, the disclosure of which is hereby expressly incorporated by reference in its entirety. Although certain examples are disclosed herein in the context of shunt structures similar to that shown in Figure 7 and described above, it should be understood that shunt structures or other implant devices integrated with pressure sensor functionality in accordance with examples of the present disclosure may have any type, form, structure, configuration, and/or may be used or configured to be used for any purpose, whether for shunting or other purpose or functionality.

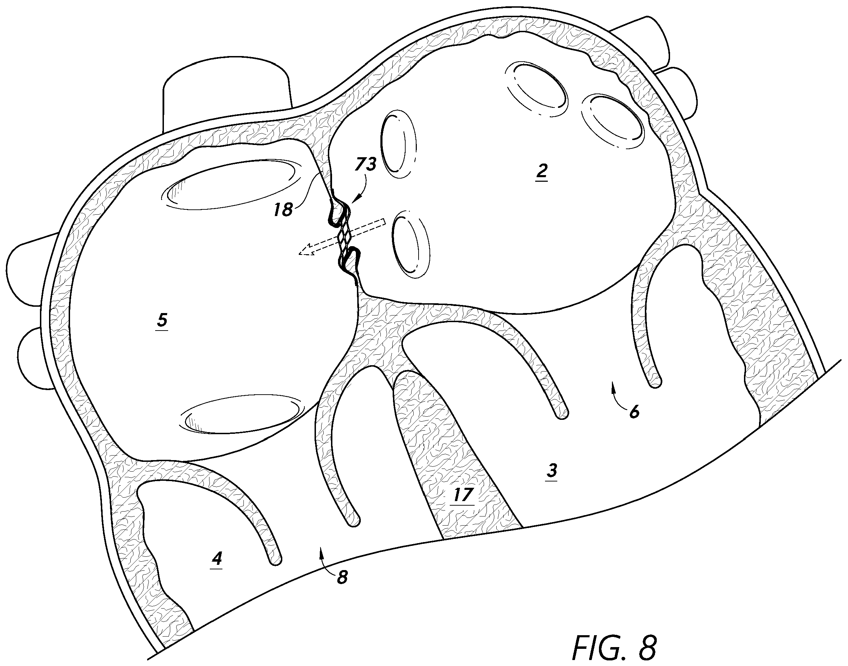

Figure 8 shows a shunt implant/anchor device/structure 73 implanted in an atrial septum 18 in accordance with one or more examples. While a shunt implant is depicted in Figure 8, the implant 73 may be any of the various implants described herein. The particular position in the interatrial septum wall 18 may be selected or determined to provide a relatively secure anchor location for the shunt structure 73. Furthermore, the shunt device/structure 73 may be implanted at a position that is desirable in consideration of future re-crossing of the septal wall 18 for future interventions. Implantation of the shunt device/structure 73 in the interatrial septum wall 18 may advantageously allow for fluid communication between the left 2 and right 5 atria.

Interatrial shunting using the shunt device/structure 73 may be well-suited for patients that are relatively highly sensitive to atrial pressure increases. For example, as pressure increases in the ventricles and/or atria and is applied against the myocardial cells, the muscles of the heart may generally be prone to contract relatively harder to process the excess blood. Therefore, as the ventricle dilates or stretches, for patients with compromised contractility of the ventricle, such patients may become more sensitive to higher pressures in the ventricle and/or atria because the heart may be unable to adequately respond or react thereto. Furthermore, increases in left atrial pressure can results in dyspnea, and therefore reduction in left atrial pressure to reduce dyspnea and/or reduce incidences of hospital readmission may be desirable through interatrial shunting. For example, when the ventricle experiences dysfunction such that is unable to accommodate build-up in fluid pressure, such fluid may backup into the atria, thereby increasing atrial pressure. With respect to heart failure, minimization of left ventricular end-diastolic pressure may be paramount. Because left ventricular end-diastolic pressure can be related to left atrial pressure, backup of fluid in the atrium can cause backup of fluid in the lungs, thereby causing undesirable and/or dangerous fluid buildup in the lungs. Interatrial shunting, such as using shunt devices in accordance with examples of the present disclosure, can divert extra fluid in the left atrium to the right atrium, which may be able to accommodate the additional fluid due to the relatively high compliance in the right atrium.

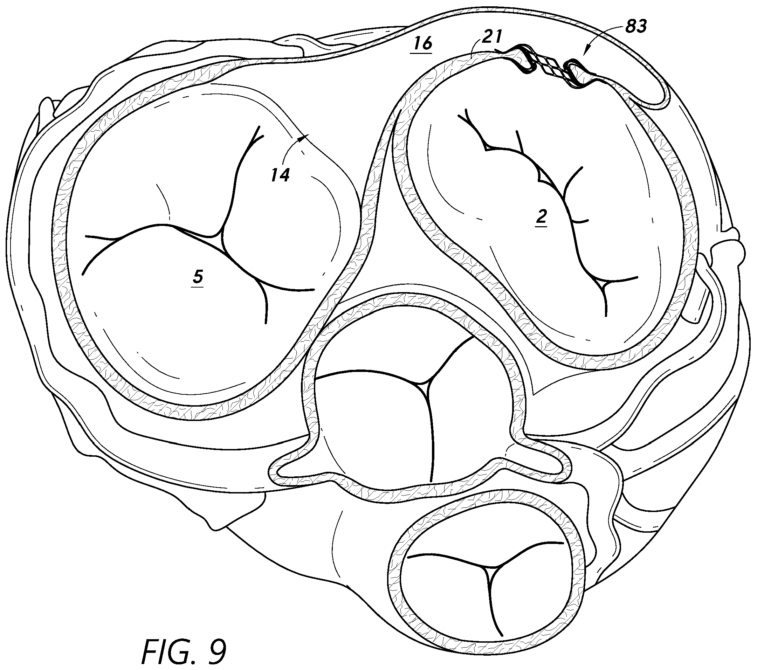

In some implementations, shunt device/structure in accordance with examples of the present disclosure may be implanted in a wall separating the coronary sinus from the left atrium, such that interatrial shunting may be achieved through the coronary sinus. Figure 9 shows a shunt device/structure 83 implanted in a tissue wall 21 between the coronary sinus 16 and the left atrium 2. While a shunt implant is depicted in Figure 9, the implant 83 may be any of the various implants described herein. Figure 9, as well as a number of the following figures, shows a section of the heart from a top-down, superior perspective with the posterior aspect oriented at the top of the page.

In some cases, left-to-right shunting through implantation of the shunt device 83 in the wall 21 between the left atrium 2 and the coronary sinus 16 can be preferable to shunting through the interatrial septum. For example, shunting through the coronary sinus 16 can provide reduced risk of thrombus and embolism. The coronary sinus is less likely to have thrombus/emboli present for several reasons. First, the blood draining from the coronary vasculature into the right atrium 5 has just passed through capillaries, so it is essentially filtered blood. Second, the ostium 14 of the coronary sinus in the right atrium is often partially covered by a pseudo-valve called the Thebesian Valve (not shown). The Thebesian Valve is not always present, but some studies show it is present in most hearts and can block thrombus or other emboli from entering in the event of a spike in right atrium pressure. Third, the pressure gradient between the coronary sinus and the right atrium into which it drains is generally relatively low, such that thrombus or other emboli in the right atrium is likely to remain there. Fourth, in the event that thrombus/emboli do enter the coronary sinus, there will be a much greater gradient between the right atrium and the coronary vasculature than between the right atrium and the left atrium. Most likely, thrombus/emboli would travel further down the coronary vasculature until right atrium pressure returned to normal and then the emboli would return directly to the right atrium.

Some additional advantages to locating the shunt structure 83 between the left atrium and the coronary sinus is that this anatomy is generally more stable than the interatrial septal tissue. By diverting left atrial blood into the coronary sinus, sinus pressures may increase by a small amount. This would cause blood in the coronary vasculature to travel more slowly through the heart, increasing perfusion and oxygen transfer, which can be more efficient and also can help a dying heart muscle to recover. In addition, by implanting the shunt device/structure 83 in the wall of the coronary sinus, damage to the interatrial septum 18 may be prevented. Therefore, the interatrial septum 18 may be preserved for later transseptal access for alternate therapies. The preservation of transseptal access may be advantageous for various reasons. For example, heart failure patients often have a number of other comorbidities, such as atrial fibrillation and/or mitral regurgitation; certain therapies for treating these conditions require a transseptal access.

It should be noted, that in addition to the various benefits of placing the implant/structure 83 between the coronary sinus 16 and the left atrium 2, certain drawbacks may be considered. For example, by shunting blood from the left atrium 2 to the coronary sinus 16, oxygenated blood from the left atrium 2 may be passed to the right atrium 5 and/or non-oxygenated blood from the right atrium 5 may be passed to the left atrium 2, both of which may be undesirable with respect to proper functioning of the heart.

### Sensor Implant Devices

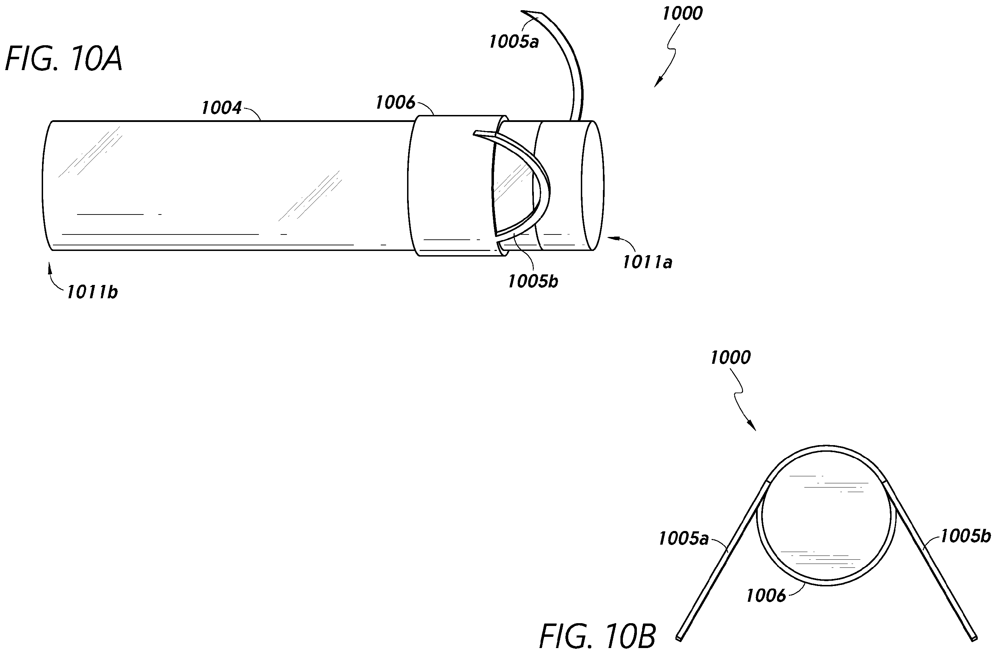

Figures 10A-10D illustrate a sensor implant device 1000 in accordance with the presently claimed invention, comprising one or more anchoring arms 1005 configured to at least partially embed into one or more tissue walls 1021, in accordance with one or more examples. The sensor implant device 1000 comprises a sensor body 1004 (i.e., sensor) having a generally cylindrical/tubular form. The sensor 1004 comprises one or more sensor components configured to provide readings and/or collect measurements relating to blood flow and/or pressure at any portion of the sensor 1004. The sensor 1004 comprises a first sensor component at or near a first end 1011a of the sensor 1004 to collect measurements at the first end 1011a. The sensor 1004 may comprise a second sensor component at or near a second end 1011b of the sensor 1004 to collect measurements at the second end 1011b. The first end 1011a may be configured to extend into and/or near a first heart chamber and/or flow pathway (e.g., the left atrium 2) and/or the second end 1011b may be configured to extend into and/or near a second heart chamber and/or flow pathway (e.g., the coronary sinus 16). In this way, the sensor 1004 may be configured to simultaneously obtain measurements for two or more different heart chambers and/or blood flow pathways.

The sensor implant device 1000 comprises one or more anchoring arms 1005, including at least a first anchoring arm 1005a and/or a second anchoring arm 1005b. While the sensor implant device 1000 is shown comprising two anchoring arms 1005, the sensor implant device 1000 can comprise any number of anchoring arms 1005. In some examples, one or more anchoring arms 1005 can extend from a sleeve 1006 attached to the sensor 1004. The sleeve 1006 may comprise a hollow circular ring configured to wrap at least partially around and/or securely attach to at least a portion of the sensor 1004. The one or more anchoring arms 1005 may be configured to attach to and/or extend from the sleeve 1006. However, the one or more anchoring arms 1005 may alternatively directly attach to and/or extend directly from the sensor 1004.

In some examples, one or more anchoring arms 1005 may have a generally curved and/or U-shaped form to facilitate embedding of the one or more anchoring arms 1005 into a tissue wall 1021 and/or to facilitate hooking of the one or more anchoring arms 1005 onto an implant 1002. For example, the one or more anchoring arms 1005 may have a generally convex form when viewed from above the first end 1011a of the sensor implant device 1000. The one or more anchoring arms 1005 comprise pointed tips to enable the one or more anchoring arms 1005 to pierce and/or puncture one or more tissue walls and/or areas of tissue within a heart. Additionally or alternatively, the one or more anchoring arms 1005 may be configured to hook onto one or more implant devices (e.g., a shunt implant 1002). In some examples, different anchoring arms 1005 may be configured to extend from the sensor 1004 at different angles. For example, as shown in Figure 10B, a first anchoring arm 1005a may be configured to extend at an approximately 45° angle with respect to a second anchoring arm 1005b when viewed from above.

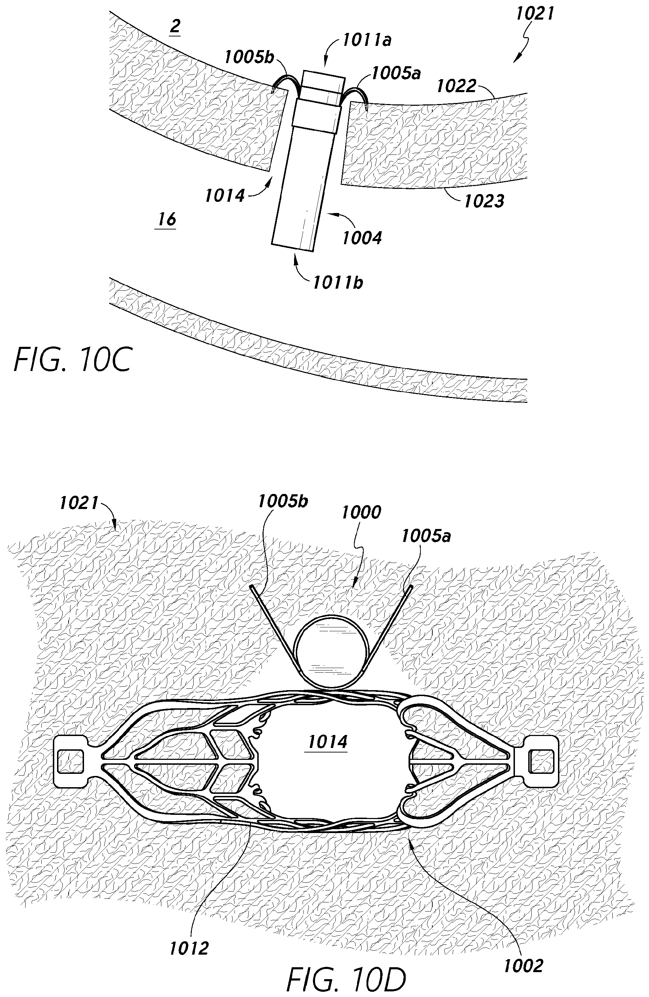

As shown in Figure 10C, the sensor implant device 1000 may be configured for placement at least partially within an opening 1014 of a tissue wall 1021. For example, at least a portion of the sensor 1004 may be configured to be situated within the opening 1014. The one or more anchoring arms 1005 may be configured to extend at least partially out of the opening 1014 and/or to puncture a first side 1022 of the tissue wall 1021. In some examples, the one or more anchoring arms 1005 may be configured to puncture and/or anchor to a left atrium 2 side (e.g., the first side 1022) of a tissue wall 1021 that separates the left atrium 2 and the coronary sinus 16. While the sensor implant device 1000 is shown anchoring to the first side 1022 of the tissue wall 1021, the sensor implant device 1000 may additionally or alternatively anchor to a second side 1023 of the tissue wall 1021. The sensor implant device 1000 comprises a first set of anchoring arms 1005 (including the first anchoring arm 1005a and second anchoring arm 1005b) extending from an upper portion of the sensor 1004 (at or near the first end 1011a of the sensor 1004) and, for example, configured to anchor to the first side 1022 of the tissue wall 1021. Additionally, the sensor implant device 1000 can comprise a second set of anchoring arms 1005 including additional anchoring arms 1005 and/or an additional sleeve 1006 at and/or extending from a lower portion of the sensor 1004 (e.g., at or near the second end 1011b of the sensor 1004) to enable the sensor implant device 1000 to anchor to the second side 1023 of the tissue wall 1021.

In some examples, the sensor implant device 1000 may be configured for use independent of additional implant devices. However, as shown in Figure 10D, the sensor implant device 1000 may additionally or alternatively be utilized in combination with other implant devices, which can include one or more shunt implants 1002. The shunt implant 1002 can comprise a barrel portion 1012 (i.e., central flow portion) configured to form and/or maintain a fluid conduit and/or opening 1014 through the tissue wall 1021. The shunt implant 1002 may additionally comprise anchoring arms configured to anchor to the tissue wall 1021. In some examples, the sensor implant device 1000 may be configured for placement adjacent to the barrel portion 1012 of the shunt implant 1002 and/or with within the opening 1014 of the tissue wall 1021. The one or more anchoring arms 1005 of the sensor implant device 1000 may be configured to extend away from the shunt implant 1002 and/or to independently anchor to the tissue wall 1021. For example, at least a portion of the sensor implant device 1000 (e.g., at least a portion of the sensor 1004) may be situated between at least a portion of the barrel portion 1012 of the shunt implant 1002 and an inner surface of the tissue wall 1021.

The sensor implant device 1000 may be configured to extend at least partially along an inner/inside surface of the opening 1014 of the tissue wall. The shunt implant 1002 may similarly be configured to contact and/or extend along at least some portions of the inside surface of the opening 1014. For example, the barrel portion 1012 of the shunt implant 1002 may have a generally cylindrical shape, which may approximate a circular shape of the opening 1014. With the sensor implant device 1000 situated between the inside surface of the opening 1014 and the central flow portion of the shunt implant 1002, the shunt implant 1002 may be configured to establish only a partial circle of contact with the inside surface of the opening 1014. For example, the sensor implant device 1000 may be in contact with a first side of the barrel portion 1012 of the shunt implant 1002 and/or may prevent the first side of the barrel portion 1012 of the shunt implant 1002 from contacting the inside surface of the opening 1014. Instead, the sensor implant device 1000 may be in contact with the inside surface of the opening 1014 and the barrel portion 1012 of the shunt implant 1002.

Delivery of the sensor implant device 1000 and the shunt implant 1002 can be performed during a single procedure and/or during separate procedures. For example, the sensor implant device 1000 and the shunt implant 1002 may be delivered via a single catheter and/or during a single delivery procedure. Alternatively, the sensor implant device 1000 may be delivered and/or anchored prior to the shunt implant 1002 or the shunt implant 1002 may be delivered and/or anchored prior to the sensor implant device 1000. In some examples, the sensor implant device 1000 may be anchored within the opening 1014 of the tissue wall 1021 before the shunt implant 1002 is placed and/or anchored within the opening 1014 of the tissue wall 1021.

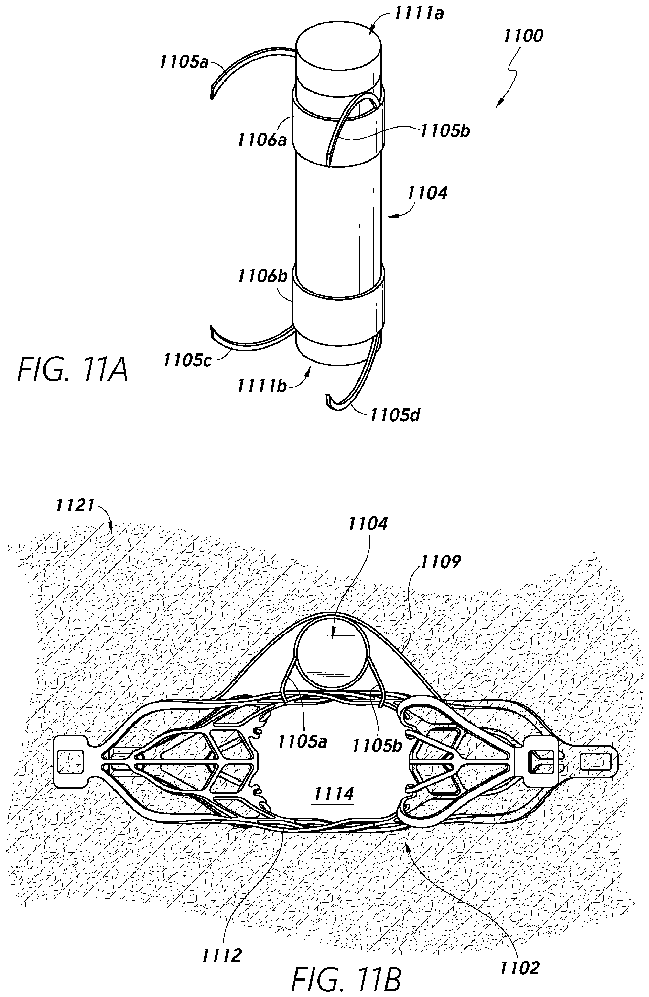

Figures 11A and 11B illustrate another sensor implant device 1100 configured to collect measurements related to blood flow within a heart, in accordance with one or more examples. The sensor implant device 1100 can comprise a sensor 1104 (i.e., sensor body) having a generally cylindrical/tubular form. The sensor 1104 may be configured to collect measurements and/or provide readings at any portion of the sensor 1104. For example, the sensor 1104 may be configured to collect measurements using one or more sensor components at a first end 1111a of the sensor 1104 and/or at a second end 1111b of the sensor 1104. The first end 1111a may be configured to extend into and/or near a first heart chamber and/or flow pathway (e.g., the left atrium 2) and/or the second end 1111b may be configured to extend into and/or near a second heart chamber and/or flow pathway (e.g., the coronary sinus 16).

The sensor implant device 1100 can comprise one or more anchoring arms 1105, which can include a first anchoring arm 1105a, a second anchoring arm 1105b, a third anchoring arm 1105c, and/or a fourth anchoring arm 1105d. While the sensor implant device 1100 is shown comprising four anchoring arms 1105, the sensor implant device 1100 can comprise any number of anchoring arms 1105. In some examples, one or more anchoring arms 1105 can extend from a first sleeve 1106a and/or a second sleeve 1106b attached to the sensor 1104. The first sleeve 1106a and/or second sleeve 1106b may comprise hollow circular rings configured to wrap at least partially around and/or securely attach to at least a portion of the sensor 1104. The one or more anchoring arms 1105 may be configured to attach to and/or extend from the first sleeve 1106a and/or the second sleeve 1106b. However, the one or more anchoring arms 1105 may alternatively directly attach to and/or extend directly from the sensor 1104.

In some examples, one or more anchoring arms 1105 may have a generally curved and/or U-shaped form to facilitate attaching to one or more implant devices (e.g., a shunt implant 1102 as shown in Figure 11B) and/or embedding of the one or more anchoring arms 1105 into a tissue wall 1121. The one or more anchoring arms 1105 may comprise hooks configured to mate with features (e.g., cells) of a shunt implant 1102 and/or similar device. Additionally or alternatively, the one or more anchoring arms 1105 may comprise pointed tips to enable the one or more anchoring arms 1105 to pierce and/or puncture one or more tissue walls 1121 and/or areas of tissue within a heart. In some examples, different anchoring arms 1105 may be configured to extend from the sensor 1104 at different angles. For example, as shown in Figure 11B, a first anchoring arm 1105a may be configured to extend at an approximately 45° angle with respect to a second anchoring arm 1105b.

The sensor implant device 1100 may be configured for placement at least partially within an opening 1114 of a tissue wall 1121. For example, at least a portion of the sensor 1104 may be configured to be situated within the opening 1114. The one or more anchoring arms 1105 may be configured to extend at least partially out of the opening 1114 and/or to couple to a barrel portion 1112 of the shunt implant 1102. In some examples, the one or more anchoring arms 1105 may be configured to couple to an upper portion of the shunt implant 1102 and/or to a lower portion of the shunt implant 1102.

In some examples, the sensor implant device 1100 may be configured for use independent of additional implant devices. However, as shown in Figure 11B, the sensor implant device 1100 may additionally or alternatively be utilized in combination with other implant devices, which can include one or more shunt implants 1102. The shunt implant 1102 can comprise a barrel portion 1112 (i.e., central flow portion) configured to form and/or maintain a fluid conduit and/or opening 1114 through the tissue wall 1121. The shunt implant 1102 may additionally comprise anchoring arms configured to anchor to the tissue wall 1121. In some examples, the sensor implant device 1100 may be configured for placement adjacent to the barrel portion 1112 of the shunt implant 1102 and/or with within the opening 1114 of the tissue wall 1121. The one or more anchoring arms 1105 of the sensor implant device 1100 may be configured to extend towards and/or couple to the shunt implant 1102 and/or to independently anchor to the tissue wall 1121. For example, at least a portion of the sensor implant device 1100 (e.g., at least a portion of the sensor 1104) may be situated between at least a portion of the barrel portion 1112 of the shunt implant 1102 and the tissue wall 1121.

Delivery of the sensor implant device 1100 and the shunt implant 1102 can be performed during a single procedure and/or during separate procedures. For example, the sensor implant device 1100 and the shunt implant 1102 may be delivered via a single catheter and/or during a single delivery procedure. Alternatively, the sensor implant device 1100 may be delivered and/or anchored prior to the shunt implant 1102 or the shunt implant 1102 may be delivered and/or anchored prior to the sensor implant device 1100. In some examples, the sensor implant device 1100 may be anchored within the opening 1114 of the tissue wall 1121 before the shunt implant 1102 is placed and/or anchored within the opening 1114 of the tissue wall 1121.

A retaining device 1109 may be used to prevent the sensor 1104 from becoming dislodged from the shunt implant 1102 after the sensor 1104 is attached to the implant 1102. For example, the retaining device 1109 can comprise one or more cords and/or wires configured to wrap at least partially around at least a portion of the sensor 1104 and/or to press the sensor 1104 toward the implant 1102. The retaining device 1109 can extend from the implant 1102 and/or sensor 1104 and/or may be configured to attach to the implant 1102 and/or sensor 1104.

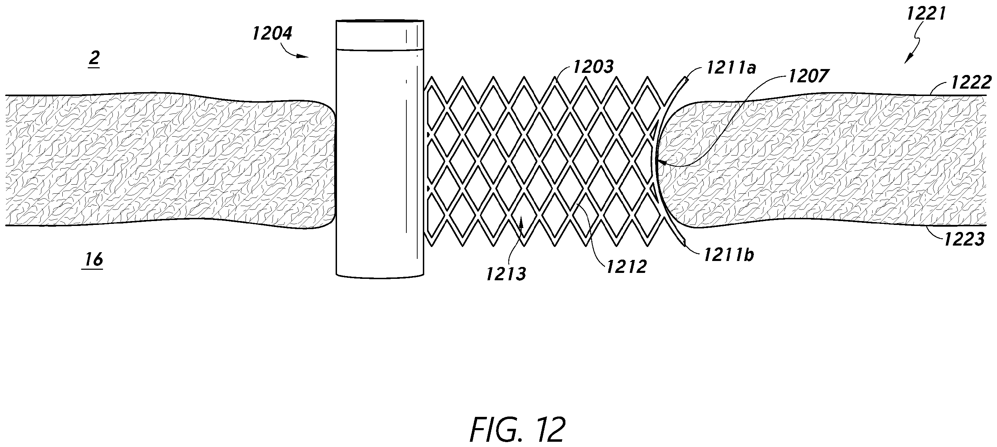

Figure 12 illustrates another sensor implant device configured to collect measurements related to blood flow within a heart, in accordance with one or more examples. The sensor implant device may comprise a sensor 1204 and/or an implant 1203 (e.g., a shunt implant). The sensor 1204 and implant 1203 may be separate and/or distinct devices. For example, the sensor 1204 may not be directly and/or indirectly coupled to the implant 1203. The implant 1203 may have a generally tubular form and/or may have an hourglass shape. For example, the implant may have a generally curved form in which a barrel portion 1207 (i.e., central flow portion) of the implant 1203 has a smaller diameter than at a first end 1211a and/or a second end 1211b of the implant 1203.

The implant 1203 may be configured to form and/or maintain a fluid conduit through the opening in the tissue wall 1221. The implant 1203 may be balloon-expandable and/or may be configured to anchor to a first side 1222 and/or to a second side (i.e., the upper wall 1223 of the coronary sinus 16) of the tissue wall 1221. In some examples, the implant 1203 may comprise a barrel portion 1207 configured for placement within the opening and/or configured to form a fluid conduit through the opening. The sensor 1204 may be configured to be positioned adjacent to the implant 1203. In some examples, at least a portion of the sensor 1204 may be situated between at least a portion of the barrel portion 1207 of the implant 1203 and the tissue wall 1221.

The barrel portion 1207 of the implant 1203 may be composed of a network of struts 1212 forming one or more cells 1213. In some examples, the implant 1203 may have generally curved/non-linear side portions to enable the first end 1211a and/or the second end 1211b to extend at least partially over the tissue wall 1221 to prevent the implant 1203 from becoming dislodged from the tissue wall 1221. For example, the first end 1211a may be configured to extend at least partially over a first side 1222 of the tissue wall 1221 and/or the second end 1211b may be configured to extend at least partially over a second side 1223 of the tissue wall 1221.

In some examples, the implant 1203 may be at least partially expandable between a compressed form and an expanded form. For example, the implant 1203 may be configured to assume a compressed form (e.g., having a relatively small and/or minimal diameter) during a delivery process and/or while situated within a catheter and/or similar delivery device. The implant 1203 may be configured to naturally expand and/or expand in response to force from an expansion device (e.g., an inflatable balloon expander). In some examples, the implant 1203 may be configured to naturally expand in response to removal from a catheter and/or similar device. For example, the implant 1203 may be at least partially composed of one or more shape-memory alloys (e.g., Nitinol) and/or may be shape-set in an expanded form. Accordingly, when the implant 1203 is removed from a catheter, the implant 1203 may naturally assume the expanded form shown in Figure 12.

The implant 1203 may be configured to maintain an expanded form following expansion. For example, a balloon expander may be situated within an inner lumen of the implant 1203 and/or may be configured to press the implant 1203 outwardly to expand and/or increase a diameter of the implant 1203. During expansion, the various struts 1212 forming the implant 1203 may separate to some extent and/or the cells 1213 may increase in width and/or decrease in length. The implant 1203 may have a generally non-elastic form such that, following expansion of the implant 1203, the implant 1203 may not naturally compress and/or may rigidly maintain the expanded form shown in Figure 12. As the implant 1203 expands, the implant 1203 may press against the sensor 1204 and/or may pin the sensor 1204 against an inner surface of the opening of the tissue wall 1221.

While the sensor 1204 is shown without anchoring arms and/or other anchoring features extending from the sensor 1204, the sensor 1204 may additionally or alternatively comprise one or more anchoring arms and/or other anchoring features to facilitate anchoring of the sensor 1204 to the tissue wall 1221 and/or to the implant 1203. For example, the sensor 1204 may comprise one or more sets of anchoring arms having various features (e.g., pointed tips, hooks, etc.) configured to pierce and/or penetrate the tissue wall 1221 and/or to hook onto the struts 1212 of the implant 1203. In some examples, the sensor 1204 may not require anchoring features and may be configured to be pinned between the implant 1203 and an inner surface of the tissue wall 1221 by frictional force. However, the sensor 1204 may additionally or alternatively comprise anchoring features to provide a more secure attachment to the tissue wall 1221 and/or implant 1203 while the sensor 1204 is pinned between the tissue wall 1221 and the implant 1203.

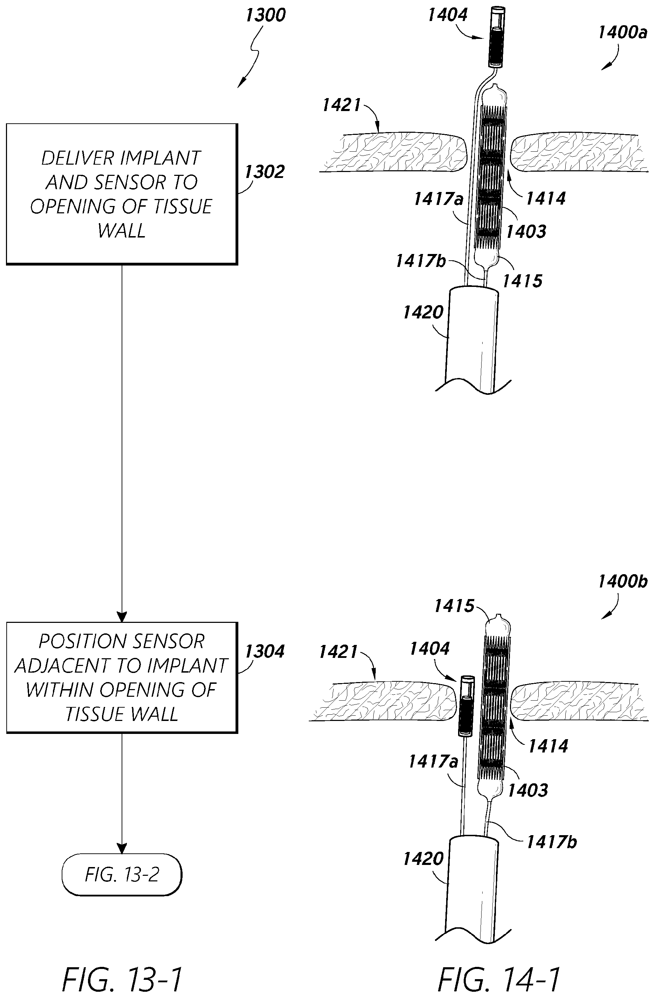

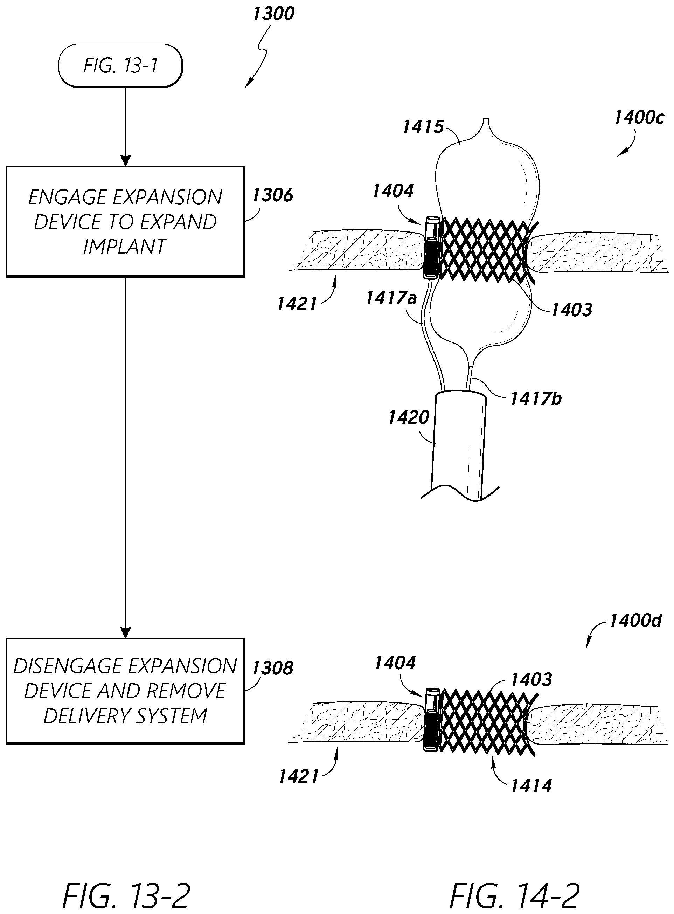

Figure 13 (Figure 13-1 and 13-2) provides a flowchart illustrating a process 1300 including one or more steps for delivering one or more implants and/or sensors to one or more target locations (e.g., an opening of a tissue wall) within a heart, in accordance with one or more examples. Figure 14 (Figures 14-1 and 14-2) provides images corresponding to steps of the process 1300 of Figure 13.

At step 1302, the process 1300 involves delivering one or more implants 1403 (e.g., the implant 1203 of Figure 12) and/or one or more sensors 1404 to an opening 1414 at a tissue wall 1421, as shown in image 1400a of Figure 14. An implant 1403 and/or sensor 1404 may be delivered simultaneously via a catheter 1420 or may be delivered separately during separate procedures. In some examples, the implant 1403 and sensor 1404 may be situated generally in-line and/or in an end-to-end orientation with each other while within the catheter 1420 and/or upon exiting the catheter 1420 to minimize the delivery profile of the implant 1403 and/or sensor 1404. The implant 1403 and sensor 1404 may be delivered along separate guidewires 1417 or a common guidewire. For example, the sensor 1404 may be delivered via a first guidewire 1417a and/or the implant 1403 may be delivered via a second guidewire 1417b.

In some examples, the implant 1403 may be positioned at least partially over an uninflated and/or unexpanded balloon 1415 and/or other expandable device. The balloon 1415 may be delivered via the second guidewire 1417b. In some examples, the implant 1403 may have a crimped and/or compressed form during delivery. For example, the implant 1403 may be crimped onto a surface of the balloon 1415. In some examples, the implant 1403 may have an increased length while in the compressed/crimped form and/or may be configured to decrease in length while laterally expanding.

At step 1304, the process 1300 involves positioning the sensor 1404 adjacent to and/or side-by-side with at least a portion of the implant 1403 and/or at least partially within the opening 1414 of the tissue wall 1421, as shown in image 1400b of Figure 14. For example, the sensor 1404 may be delivered ahead of and/or in front of the implant 1403 and/or may be pulled back and/or to the side to position the sensor 1404 adjacent to and/or in a side-by-side orientation with the implant 1403. Alternatively, the implant 1403 may be delivered ahead of and/or in front of the sensor 1404 and/or may be pulled back and/or to the side to position the implant 1403 adjacent to the sensor 1404.

At step 1306, the process 1300 involves engaging, inflating, and/or expanding the balloon 1415 to cause corresponding lateral expansion of the implant 1403, as shown in image 1400c of Figure 14. For example, expansion of the implant 1403 may cause the implant 1403 to press against sides of the opening 1414. In some example, the implant 1403 may be configured to decrease in length while expanding laterally as a result of modified positioning and/or orientation of various struts and/or cells forming the implant 1403. Expansion of the implant 1403 may cause the implant 1403 to press against the sensor 1404 and/or to press the sensor 1404 against a side of the opening 1414. Accordingly, the sensor 1404 may be securely pinned between at least a portion of the implant 1403 and at least a portion of tissue forming a side of the opening 1414.

At step 1308, the process 1300 involves disengaging, deflating, and/or compressing the balloon 1415 and/or removing the balloon 1415 and/or other delivery system devices, as shown in image 1400d of Figure 14. The implant 1403 may be configured to maintain an expanded form following removal and/or compression of the balloon 1415.

In accordance with some implementations of the present disclosure, a sensor implant system comprises a shunt body comprising a central flow portion configured for placement at least partially within an opening of a tissue wall and a sensor implant device comprising a sensor body. The sensor implant device is configured for placement within the opening of the tissue wall and between the central flow portion of the shunt body and an inner wall of the opening of the tissue wall.

The sensor implant device may comprise one or more arms coupled to the sensor body. In some examples, the one or more arms are configured to pierce the tissue wall.

In some examples, the one or more arms are configured to attach to the central flow portion of the shunt body. The sensor implant device may comprise a first set of two arms extending from an upper portion of the sensor body and a second set of two arms extending from a lower portion of the sensor body.

The one or more arms may be generally curved. In some examples, the one or more arms extend from an upper portion of the sensor body, and wherein the one or more arms have a convex curvature when viewed from above the upper portion of the sensor body.

In some examples, the sensor implant device further comprises a sleeve encircling at least portion of the sensor body, and wherein the one or more arms extend from the sleeve. The one or more arms may extend generally laterally from the sensor body.

The one or more arms may extend at an approximately 90-degree angle from each other. In some examples, the one or more arms are configured to extend towards the shunt body.

In some examples, the one or more arms are configured to extend away from the shunt body. The shunt body may be expandable from a compressed form to an expanded form.

The shunt body may be configured to maintain the expanded form following expansion. In some examples, the shunt body comprises a network of struts forming generally diamond shaped cells.

In some examples, the shunt body has a generally cylindrical shape. The shunt body may have an hourglass shape in which a midsection of the shunt body is configured for placement within the opening of the tissue wall and wherein the shunt body comprises a flared first end and a flared second end configured to extend out of the opening of the tissue wall.

The sensor implant device may comprise a first sensor component configured to obtain measurements at a first side of the tissue wall and a second sensor component configured to obtain measurements at a second side of the tissue wall. In some examples, the shunt body comprises a retaining device configured to wrap at least partially around the sensor body.

In some examples, the shunt body and the sensor implant device are separate devices. The shunt body may comprise one or more anchoring arms.

Some implementations of the present disclosure relate to a method comprising percutaneously delivering a shunt body to an opening of a tissue wall. The shunt body comprises a central flow portion. The method further comprises percutaneously delivering a sensor implant device comprising a sensor body to the opening in the tissue wall and positioning the central flow portion and the sensor body in a side-by-side orientation within the opening.

In some examples, the sensor implant device further comprises one or more arms extending from the sensor body. The method may further comprise anchoring the sensor implant device to the shunt body using the one or more arms.

The method may further comprise anchoring the sensor implant device to the tissue wall using the one or more arms. In some examples, the method further comprises expanding the shunt body to pin the sensor implant device between the shunt body and an inner wall of the opening.

In some examples, the method further comprises positioning the shunt body and the sensor implant device in an end-to-end orientation within a catheter.

In accordance with some implementations of the present disclosure, a sensor implant device comprises a sensor body comprising one or more sensor components configured to provide sensor readings relating to one or more physiological parameters and one or more anchoring arms extending from the sensor body.

At least some of the one or more anchoring arms may have pointed ends for puncturing a tissue wall. In some examples, at least some of the one or more anchoring arms are configured to hook onto a central flow portion of a shunt body.

In some examples, the sensor body comprises a first sensor component at an upper portion of the sensor body and a second sensor component at a lower portion of the sensor body. The sensor implant device may comprise a first set of two arms extending from an upper portion of the sensor body and a second set of two arms extending from a lower portion of the sensor body.

The one or more anchoring arms may be generally curved. In some examples, the one or more anchoring arms extend from an upper portion of the sensor body. The one or more anchoring arms may have a convex curvature when viewed from above the upper portion of the sensor body.

In some examples, the sensor implant device further comprises a sleeve encircling at least portion of the sensor body. The one or more anchoring arms may extend from the sleeve.

The one or more anchoring arms may extend generally laterally from the sensor body. In some examples, the one or more anchoring arms extend at an approximately 90-degree angle from each other.

### Additional Examples

Depending on the example, certain acts, events, or functions of any of the processes or algorithms described herein can be performed in a different sequence, may be added, merged, or left out altogether. Thus, in certain examples, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain examples include, while other examples do not include, certain features, elements and/or steps. Thus, such conditional language is not generally intended to imply that features, elements and/or steps are in any way required for one or more examples or that one or more examples necessarily include logic for deciding, with or without author input or prompting, whether these features, elements and/or steps are included or are to be performed in any particular example. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain examples require at least one of X, at least one of Y and at least one of Z to each be present.

It should be appreciated that in the above description of examples, various features are sometimes grouped together in a single example, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular example herein can be applied to or used with any other example(s). Further, no component, feature, step, or group of components, features, or steps are necessary or indispensable for each example. Thus, it is intended that the scope of the inventions herein disclosed and claimed below should not be limited by the particular examples described above, but should be determined only by a fair reading of the claims that follow.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). **In** addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example examples belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. A sensor implant device (1000) comprising:
a sensor body (1004) comprising one or more sensor components configured to provide sensor readings relating to blood flow and/or pressure, wherein the sensor body (1004) has a generally cylindrical form, and wherein the one or more sensor components comprise a first sensor component at or near a first end (1011a) of the sensor body (1004); and
one or more anchoring arms (1005) extending from the sensor body, wherein the one or more anchoring arms (1005) comprise a first set of anchoring arms (1005a, 1005b) extending from the sensor body (1004) at or near the first end (1011a) of the sensor body (1004), and wherein at least some of the one or more anchoring arms have pointed ends for puncturing a tissue wall (1021).

2. The sensor implant device of claim 1, wherein at least some of the one or more anchoring arms are configured to hook onto a central flow portion (1012) of a shunt body (1002).

3. The sensor implant device of claim 1 or claim 2, wherein the sensor body comprises a second sensor component at or near a second end (1011b) of the sensor body.

4. The sensor implant device of any of claims 1-3, wherein the sensor implant device comprises a first set of two arms (1005a, 1005b, 1105a, 1105b) extending from the sensor body at or near the first end (1011a) of the sensor body and a second set of two arms (1105c, 1105d) extending from the sensor body at or near a second end (1011b) of the sensor body.

5. The sensor implant device of any of claims 1-4, wherein the one or more anchoring arms are generally curved.

6. The sensor implant device of claim 5, wherein the one or more anchoring arms extend from the sensor body at or near the first end (1011a) of the sensor body, and wherein the one or more anchoring arms have a convex curvature when viewed from above the first end (1011a) of the sensor body.

7. The sensor implant device of any of claims 1-6, further comprising a sleeve (1006, 1106a, 1106b) encircling at least portion of the sensor body, and wherein the one or more anchoring arms extend from the sleeve.

8. The sensor implant device of any of claims 1-7, wherein the one or more anchoring arms extend
a) generally laterally from the sensor body; and/or
b) at an approximately 90-degree angle from each other.

9. A sensor implant system comprising:
a shunt body (1002) comprising a central flow portion (1012) configured for placement at least partially within an opening of a tissue wall (1021); and
a sensor implant device (1000) comprising a sensor body, the sensor implant device configured for placement within the opening of the tissue wall and between the central flow portion of the shunt body and an inner wall of the opening of the tissue wall, wherein the sensor implant device comprises a sensor implant device (1000) according to any one of claims 1 to 8.

10. The sensor implant system of claim 9, wherein the one or more arms are configured to extend towards the shunt body.

11. The sensor implant system of any of claims 9 or 10, wherein the one or more arms are configured to extend away from the shunt body.

12. The sensor implant system of any of claims 9-11, wherein the shunt body is expandable from a compressed form to an expanded form,
optionally wherein the shunt body is configured to maintain the expanded form following expansion.

13. The sensor implant system of claim 12, wherein the shunt body:
a) comprises a network of struts (1212) forming generally diamond shaped cells (1213);
b) has a generally cylindrical shape; and/or
c) has an hourglass shape in which a midsection of the shunt body is configured for placement within the opening of the tissue wall and wherein the shunt body comprises a flared first end and a flared second end configured to extend out of the opening of the tissue wall.

14. The sensor implant system of any of claims 9-13, wherein the sensor implant device comprises a first sensor component configured to obtain measurements at a first side of the tissue wall and a second sensor component configured to obtain measurements at a second side of the tissue wall.

15. The sensor implant system of any of claims 9-14, wherein:
a) the shunt body comprises a retaining device (1109) configured to wrap at least partially around the sensor body;
b) the shunt body and the sensor implant device are separate devices; and/or
c) the shunt body comprises one or more anchoring arms.

## Patentansprüche

1. Sensorimplantatvorrichtung (1000), die Folgendes umfasst:
einen Sensorkörper (1004), der eine oder mehrere Sensorkomponenten umfasst, die dazu ausgelegt sind, Sensormesswerte in Bezug auf Blutfluss und/oder Druck bereitzustellen, wobei der Sensorkörper (1004) eine allgemein zylindrische Form aufweist und wobei die eine oder die mehreren Sensorkomponenten eine erste Sensorkomponente an oder nahe einem ersten Ende (1011a) des Sensorkörpers (1004) umfassen; und
einen oder mehrere Verankerungsarme (1005), die sich von dem Sensorkörper erstrecken, wobei der eine oder die mehreren Verankerungsarme (1005) einen ersten Satz von Verankerungsarmen (1005a, 1005b) umfassen, die sich von dem Sensorkörper (1004) an oder nahe dem ersten Ende (1011a) des Sensorkörpers (1004) erstrecken, und wobei zumindest einige des einen oder der mehreren Verankerungsarme spitze Enden zum Punktieren einer Gewebewand (1021) aufweisen.

2. Sensorimplantatvorrichtung nach Anspruch 1, wobei zumindest einige des einen oder der mehreren Verankerungsarme dazu konfiguriert sind, sich auf einen zentralen Strömungsabschnitt (1012) eines Shuntkörpers (1002) einzuhaken.

3. Sensorimplantatvorrichtung nach Anspruch 1 oder Anspruch 2, wobei der Sensorkörper eine zweite Sensorkomponente an oder nahe einem zweiten Ende (1011b) des Sensorkörpers umfasst.

4. Sensorimplantatvorrichtung nach einem der Ansprüche 1-3, wobei die Sensorimplantatvorrichtung einen ersten Satz von zwei Armen (1005a, 1005b, 1105a, 1105b), die sich von dem Sensorkörper an oder nahe dem ersten Ende (1011a) des Sensorkörpers erstrecken, und einen zweiten Satz von zwei Armen (1105c, 1105d), die sich von dem Sensorkörper an oder nahe einem zweiten Ende (1011b) des Sensorkörpers erstrecken, umfasst.

5. Sensorimplantatvorrichtung nach einem der Ansprüche 1-4, wobei der eine oder die mehreren Verankerungsarme allgemein gekrümmt sind.

6. Sensorimplantatvorrichtung nach Anspruch 5, wobei sich der eine oder die mehreren Verankerungsarme von dem Sensorkörper an oder nahe dem ersten Ende (1011a) des Sensorkörpers erstrecken und wobei der eine oder die mehreren Verankerungsarme eine konvexe Krümmung aufweisen, wenn sie von oberhalb des ersten Endes (1011a) des Sensorkörpers betrachtet werden.

7. Sensorimplantatvorrichtung nach einem der Ansprüche 1-6, ferner umfassend eine Hülse (1006, 1106a, 1106b), die mindestens einen Teil des Sensorkörpers umgibt, und wobei sich der eine oder die mehreren Verankerungsarme von der Hülse erstrecken.

8. Sensorimplantatvorrichtung nach einem der Ansprüche 1-7, wobei sich der eine oder die mehreren Verankerungsarme erstrecken
a) allgemein seitlich von dem Sensorkörper aus; und/oder
b) in einem Winkel von etwa 90 Grad zueinander.

9. Sensorimplantatsystem, das Folgendes umfasst:
einen Shuntkörper (1002), der einen zentralen Strömungsabschnitt (1012) umfasst, der zur Platzierung zumindest teilweise innerhalb einer Öffnung einer Gewebewand (1021) konfiguriert ist; und
eine Sensorimplantatvorrichtung (1000), die einen Sensorkörper umfasst, wobei die Sensorimplantatvorrichtung zur Platzierung innerhalb der Öffnung der Gewebewand und zwischen dem zentralen Strömungsabschnitt des Shuntkörpers und einer Innenwand der Öffnung der Gewebewand konfiguriert ist, wobei die Sensorimplantatvorrichtung eine Sensorimplantatvorrichtung (1000) nach einem der Ansprüche 1 bis 8 umfasst.

10. Sensorimplantatsystem nach Anspruch 9, wobei der eine oder die mehreren Arme dazu konfiguriert sind, sich hin zum Shuntkörper zu erstrecken.

11. Sensorimplantatsystem nach einem der Ansprüche 9 oder 10, wobei der eine oder die mehreren Arme dazu konfiguriert sind, sich von dem Shuntkörper weg zu erstrecken.

12. Sensorimplantatsystem nach einem der Ansprüche 9-11, wobei der Shuntkörper von einer komprimierten Form zu einer expandierten Form expandierbar ist,
wobei optional der Shuntkörper dazu konfiguriert ist, die expandierte Form nach der Expansion beizubehalten.

13. Sensorimplantatsystem nach Anspruch 12, wobei der Shuntkörper:
a) ein Netzwerk von Streben (1212) umfasst, die allgemein rautenförmige Zellen (1213) bilden;
b) eine im Allgemeinen zylindrische Form aufweist; und/oder
c) eine Sanduhrform aufweist, bei der ein Mittelabschnitt des Shuntkörpers zur Platzierung innerhalb der Öffnung der Gewebewand konfiguriert ist und wobei der Shuntkörper ein aufgeweitetes erstes Ende und ein aufgeweitetes zweites Ende umfasst, die dazu konfiguriert sind, sich aus der Öffnung der Gewebewand heraus zu erstrecken.

14. Sensorimplantatsystem nach einem der Ansprüche 9-13, wobei die Sensorimplantatvorrichtung eine erste Sensorkomponente, die dazu ausgelegt ist, Messungen an einer ersten Seite der Gewebewand zu erhalten, und eine zweite Sensorkomponente, die dazu ausgelegt ist, Messungen an einer zweiten Seite der Gewebewand zu erhalten, umfasst.

15. Sensorimplantatsystem nach einem der Ansprüche 9-14, wobei:
a) der Shuntkörper eine Haltevorrichtung (1109) umfasst, die dazu ausgelegt ist, den Sensorkörper zumindest teilweise zu umwickeln;
b) der Shuntkörper und die Sensorimplantatvorrichtung separate Vorrichtungen sind; und/oder
c) der Shuntkörper einen oder mehrere Verankerungsarme umfasst.

## Revendications

1. Dispositif d'implant (1000) de capteur, comprenant :
un corps (1004) de capteur comprenant un ou plusieurs composants de capteur configurés pour fournir des lectures de capteur relatives au débit sanguin et/ou à la pression sanguine, le corps (1004) de capteur présentant une forme généralement cylindrique et ledit un ou lesdits plusieurs composants de capteur comprenant un premier composant de capteur au niveau ou à proximité d'une première extrémité (1011a) du corps (1004) de capteur ; et
un ou plusieurs bras d'ancrage (1005) s'étendant à partir du corps de capteur, ledit un ou lesdits plusieurs bras d'ancrage (1005) comprenant un premier ensemble de bras d'ancrage (1005a, 1005b) s'étendant à partir du corps (1004) de capteur au niveau ou à proximité de la première extrémité (1011a) du corps (1004) de capteur et au moins certains bras parmi ledit un ou lesdits plusieurs bras d'ancrage présentant des extrémités pointues destinées à perforer une paroi tissulaire (1021).

2. Dispositif d'implant de capteur selon la revendication 1, au moins certains bras parmi ledit un ou lesdits plusieurs bras d'ancrage étant conçus pour s'accrocher sur une partie d'écoulement centrale (1012) d'un corps de dérivation (1002).

3. Dispositif d'implant de capteur selon la revendication 1 ou la revendication 2, le corps de capteur comprenant un second composant de capteur au niveau ou à proximité d'une seconde extrémité (1011b) du corps de capteur.

4. Dispositif d'implant de capteur selon l'une quelconque des revendications 1 à 3, le dispositif d'implant de capteur comprenant un premier ensemble de deux bras (1005a, 1005b, 1105a, 1105b) s'étendant à partir du corps de capteur au niveau ou à proximité de la première extrémité (1011a) du corps de capteur et un second ensemble de deux bras (1105c, 1105d) s'étendant à partir du corps de capteur au niveau ou à proximité d'une seconde extrémité (1011b) du corps de capteur.

5. Dispositif d'implant de capteur selon l'une quelconque des revendications 1 à 4, ledit un ou lesdits plusieurs bras d'ancrage étant généralement incurvés.

6. Dispositif d'implant de capteur selon la revendication 5, ledit un ou lesdits plusieurs bras d'ancrage s'étendant à partir du corps de capteur au niveau ou à proximité de la première extrémité (1011a) du corps de capteur et ledit un ou lesdits plusieurs bras d'ancrage présentant une courbure convexe lorsqu'ils sont vus depuis le dessus de la première extrémité (1011a) du corps de capteur.

7. Dispositif d'implant de capteur selon l'une quelconque des revendications 1 à 6, comprenant en outre un manchon (1006, 1106a, 1106b) entourant au moins une partie du corps de capteur et ledit un ou lesdits plusieurs bras d'ancrage s'étendant à partir du manchon.

8. Dispositif d'implant de capteur selon l'une quelconque des revendications 1 à 7, ledit un ou lesdits plusieurs bras d'ancrage s'étendant
a) généralement latéralement à partir du corps de capteur ; et/ou
b) selon un angle d'environ 90 degrés les uns des autres.

9. Système d'implant de capteur, comprenant :
un corps de dérivation (1002) comprenant une partie d'écoulement centrale (1012) conçue pour être placée au moins partiellement dans une ouverture d'une paroi tissulaire (1021) ; et
un dispositif d'implant (1000) de capteur comprenant un corps de capteur, le dispositif d'implant de capteur étant conçu pour être placé dans l'ouverture de la paroi tissulaire et entre la partie d'écoulement centrale du corps de dérivation et une paroi interne de l'ouverture de la paroi tissulaire, le dispositif d'implant de capteur comprenant un dispositif d'implant (1000) de capteur selon l'une quelconque des revendications 1 à 8.

10. Système d'implant de capteur selon la revendication 9, ledit un ou lesdits plusieurs bras étant configurés pour s'étendre vers le corps de dérivation.

11. Système d'implant de capteur selon l'une quelconque des revendications 9 ou 10, ledit un ou lesdits plusieurs bras étant configurés pour s'étendre en s'éloignant du corps de dérivation.

12. Système d'implant de capteur selon l'une quelconque des revendications 9 à 11, le corps de dérivation pouvant être déployé à partir d'une forme comprimée vers une forme déployée,
éventuellement, le corps de dérivation étant configuré pour maintenir la forme déployée après le déploiement.

13. Système d'implant de capteur selon la revendication 12, le corps de dérivation :
a) comprenant un réseau d'entretoises (1212) formant des cellules (1213) généralement en forme de losange ;
b) présentant une forme généralement cylindrique ; et/ou
c) présentant une forme de sablier dans laquelle une section médiane du corps de dérivation est conçue être placée dans l'ouverture de la paroi tissulaire et le corps de dérivation comprenant une première extrémité évasée et une seconde extrémité évasée conçues pour s'étendre hors de l'ouverture de la paroi tissulaire.

14. Système d'implant de capteur selon l'une quelconque des revendications 9 à 13, le dispositif d'implant de capteur comprenant un premier composant de capteur configuré pour obtenir des mesures au niveau d'un premier côté de la paroi tissulaire et un second composant de capteur configuré pour obtenir des mesures au niveau d'un second côté de la paroi tissulaire.

15. Système d'implant de capteur selon l'une quelconque des revendications 9 à 14 :
a) le corps de dérivation comprenant un dispositif de retenue (1109) conçu pour s'enrouler au moins partiellement autour du corps de capteur ;
b) le corps de dérivation et le dispositif d'implant de capteur étant des dispositifs séparés ; et/ou
c) le corps de dérivation comprenant un ou plusieurs bras d'ancrage.
